# EUROPÄISCHE PATENTSCHRIFT

(11) **EP 2 841 412 B1**
(45) Veröffentlichungstag und Bekanntmachung des Hinweises auf die Patenterteilung: **06.04.2016**
(21) Anmeldenummer: 13719491.6
(22) Anmeldetag: 24.04.2013
(51) Int. Cl.: C07C 201/16

(54) **VERFAHREN ZUR WÄSCHE VON DINITROTOLUOL**
METHOD FOR WASHING DINITROTOLUENE
PROCÉDÉ DESTINÉ AU LAVAGE DU DINITROTOLUOL

(30) Priorität: 25.04.2012 EP 12165511
(43) Veröffentlichungstag der Anmeldung: 04.03.2015
(73) Patentinhaber: BASF SE, 67056 Ludwigshafen am Rhein (DE); Josef Meissner GmbH & Co. KG, 50968 Köln (DE)
(72) Erfinder: NETO, Samuel, 68159 Mannheim (DE); FRITZ, Rüdiger, 02994 Bernsdorf (DE); HEMPEL, Renate, 01945 Ruhland (DE); ALLARDT, Holger, 01987 Schwarzheide (DE); BECKER, Barbara, 69509 Mörlenbach (DE); LESCHINSKI, Julia, B-1050 Ixelles (BE); AHRENS, Sebastian, 69168 Wiesloch (DE); HERMANN, Heinrich, 50858 Köln (DE); HAENDEL, Mirko, 53819 Neunkirchen-Seelscheid (DE); POEHLMANN, Juergen, 50969 Köln (DE)
(74) Vertreter: Schuck, Alexander
(86) Internationale Anmeldenummer: PCT/EP2013/058530
(87) Internationale Veröffentlichungsnummer: WO 2013/160367

(56) Entgegenhaltungen:
- EP-A1- 0 736 514
- EP-A1- 1 780 195
- CA-A1- 1 034 603
- DE-B3-102006 013 579

## Beschreibung

Die Erfindung betrifft ein Verfahren zur Wäsche eines bei der Nitrierung von Toluol nach Abtrennung der Nitriersäure anfallenden Rohgemischs enthaltend Dinitrotoluol (DNT), Salpetersäure, Stickstoffoxide und Schwefelsäure.

Die vorliegende Erfindung betrifft insbesondere ein Verfahren zur Wäsche des DNT enthaltenden Rohgemischs (nachfolgend auch als Roh-DNT bezeichnet) aus einer kontinuierlichen isothermen Nitrierung von Toluol zu DNT in einem Gemisch von Schwefel- und Salpetersäure, das es gestattet, das resultierende Waschwasser aus dieser Wäsche direkt oder nach Entfernung des in diesem Wasser gelösten DNT durch Extraktion, ohne weitere physikalische oder chemische Vorbehandlung, einer biologischen Behandlung in einer Kläranlage zu unterwerfen, wobei das aus der Kläranlage ablaufende Abwasser die Anforderungen bezüglich Toxizität, wie sie z.B. in der Abwasserverordnung der Bundesrepublik Deutschland, Anhang 22 : Chemische Industrie, vorgegeben sind, erfüllt.

Bei der kontinuierlichen isothermen oder adiabatischen Nitrierung von Toluol zu DNT in einer oder zwei Stufen im Gegenstrom mit Mischsäure wird nach Phasentrennung stets ein Rohprodukt an Nitroaromat erhalten, das vor Weiterverwendung von den darin gelösten Verunreinigungen befreit werden muss. Neben der im Nitroaromat gelösten bzw. als Mikroemulsion vorliegenden Nitrierendsäure aus Salpetersäure, Schwefelsäure und Stickoxiden sind noch Oxidationsprodukte aus Nebenreaktionen mit dem zu nitrierenden Aromaten, wie Mono-, Di- und Trinitrokresole, oder aromatischen Carbonsäuren, wie z.B. Mono- und Dinitrobenzoesäuren (im Folgenden als Nitrobenzoesäuren oder NBS bezeichnet) und deren Abbauprodukte, im Roh-DNT enthalten.

Gemäß dem Stand der Technik werden diese Verunreinigungen aus dem Rohprodukt durch eine mehrstufige Wäsche entfernt, bevor der Nitroaromat einer direkten Verwendung, einer Isomerentrennung oder einer Hydrierung zu den entsprechenden Aminen zugeführt wird.

Üblicherweise (siehe z.B. F. Meissner et al in Industrial and Engineering Chemistry Vol 46, 721 (1954), Ullmanns Enzyklopädie der technischen Chemie, 4. Auflage. Bd.17, S. 385-386; H. Hermann et al in ACS-Symposium Series 632, 238 (1996) Seite 241 ;ed. L.F. Albright, R.V.C Carr, R.J.Schmitt, US 6 288 289, EP 1 816 117) besteht die Wäsche des rohes Nitroaromaten wie des DNT zur Entfernung der darin gelösten und suspendierten Säuren des Nitriergemisches, der Nitrokresole und anderer saurer und sonst noch mit dem Waschmittel extrahierbaren Verunreinigungen aus drei Schritten:
1. einer Wäsche zur Entfernung der gelösten und suspendierten Mineralsäuren wie Schwefelsäure, Salpetersäure und von Stickstoffoxiden (saure Wäsche);
2. einer Wäsche in Gegenwart einer Base (Alkaliwäsche) wie Natriumkarbonat (Soda), Natriumbikarbonat, Ammoniak , Natronlauge, Kalilauge etc (siehe US 4 482 769, US 4 597 875, US 6 288 289) zur Entfernung der im rohen Nitroaromaten gelösten schwach aciden Verunreinigungen wie der Nitrokresole, Nitrobenzoesäuren und Abbauprodukte aus dem oxidativen Abbau der Nitrokresole oder von aliphatischen oder cyclischen Kohlenwasserstoffen, wie z.B. Oxalsäure;
3. einer Neutralwäsche (Neutralwäsche) zur Entfernung der Alkali-Restspuren und der weiteren Reduzierung der noch in Spuren im Produkt verbliebenen Verunreinigungen. Als Waschmedium wird dafür üblicherweise Wasser eingesetzt, und die Wäsche wird als flüssig/flüssig Wäsche bei den Temperaturen, bei denen der zu waschende Nitroaromat als Flüssigkeit vorliegt, durchgeführt.

Ziel dieser Wäschen ist es, neben einem reinen Produkt möglichst wenig Abwasser pro Tonne Produkt zu erhalten, in dem die ausgewaschenen Verunreinigungen so vorliegen, dass ihre Entsorgung kostengünstig durchgeführt werden kann.

Dementsprechend fallen drei verschiedene Abwässer aus der Wäsche des rohen DNT an, nämlich das Abwasser aus der sauren, der alkalischen und der Neutralwäsche.

Das Abwasser aus der sauren Wäsche enthält im Falle von DNT, wenn z.B. nach dem in EP 0 736 514, und EP 1 780 195 beschriebenen Verfahren gearbeitet wird, im wesentlichen neben dem gelösten Produkt von bis zu 0,5 Gew.-% (entsprechend seiner Löslichkeit) die im Rohprodukt gelösten Mineralsäuren, wie Schwefelsäure, Salpetersäure und Nitrose bzw. salpetrige Säure im Mengen von 25 - 40 Gew.-% Gesamtsäure und nur Spuren der schwächer aciden Nitrophenole, Nitrokresole, Nitrobenzoesäuren oder anderen organischen Säuren, wie Oxalsäure (aus dem oxidativen Abbau durch Salpetersäure). Der TOC eines solchen Abwassers mit ca. 8-9000 mg/l ist überraschend hoch und resultiert zu weniger als 50% aus dem in der Waschsäure gelösten DNT.

Das Abwasser aus der alkalische Wäsche mit einem pH größer 9 enthält, im Falle des DNT, neben ca. 500-5000 ppm an Nitrat, 500-5000 ppm an Nitrit und bis zu einigen hundert ppm an Sulfat, im wesentlichen neben bis zu 1300 ppm des gelösten Produkts, entsprechend seiner Löslichkeit, die im Rohprodukt gelösten phenolischen Nitroverbindungen von bis zu 800 ppm an Nitrokresolen, bis zu 800 ppm an Nitrobenzoesäuren und Spuren an Cyanid aus dem oxidativen Abbau der Nitrokresole auf der DNT-Stufe (siehe US 4 361 712). Der TOC in einem solchen alkalischen Waschwasser aus einer Wäsche von DNT liegt zwischen 1500-3200 mg/l. Nur bis zu 50% dieses TOC resultieren aus den im Abwasser gelösten Nitrokresolen und NBS. Der Rest ist unbekannt und rührt wahrscheinlich von Oxidationsprodukten der Nitrokresole auf der DNT-Stufe her. Das alkalische Abwasser ist generell tief gefärbt von intensiv orange bis rotbraun.

Das Abwasser aus der Neutralwäsche kann schwach alkalisch oder schwach sauer sein mit einem pH von 3-8, je nachdem wie groß der Mitriss aus der vorhergehenden Waschstufe (der alkalischen Wäsche) ist und welche Base in der alkalischen Wäsche eingesetzt wurde. Neben dem Produkt, das, entsprechend seiner Löslichkeit bei der verwendeten Waschtemperatur bis zur Sättigung gelöst ist, enthält das Abwasser aus der Neutralwäsche nur Restspuren der zur alkalischen Wäsche eingesetzten Base und der im DNT noch verbliebenen Verunreinigungen, vor allem der Nitrokresole.

Zur Minimierung des für diese Wäsche benötigten Wassermengen erfolgt die Wäsche im Gegenstrom derart dass das zur Neutralwäsche benutzte Wasser nach Zusatz von Basen in der Alkaliwäsche eingesetzt wird (s. A.B. Quakenbush et.al., The Olin Dinitrotoluene (DNT) Process, Polyurethane World Congr. Proc.1993, Publish.: Technomic Lancaster, p. 485) oder dass bei der sauren Wäsche mit einer minimalen Menge an Wasser derart gewaschen wird, dass eine konzentrierte Säure erhalten wird, die, wie in EP 0 279 312, EP 0 736 514, und EP 1 780 195 beschrieben, direkt oder nach weiterer Aufkonzentrierung in die Nitrierung zurückgeführt werden kann. Die bei Aufkonzentrierung von Waschwässern anfallenden Brüdenkondensate werden in die jeweiligen Wäschen zurückgeführt.

Bevor die Abwässer aus einer Anlage zur Nitrierung von Toluol zu DNT, den Waschwässern aus der Wäsche des Roh-DNT und den Brüdenkondensaten aus der Aufkonzentrierung der End- und von Waschsäuren, abgegeben werden können, müssen sie aufwendig behandelt werden, um die jeweils gültigen Abgabeparameter, wie niedergelegt in der Abwasserverordnung der BRD, für z.B. CSB, Stickstoff, Schwermetalle und biologischen Parameter vor Einleitung in einen Vorfluter zu erreichen.

Als behandlungsbedürftige Abwässer aus Nitrieranlagen fallen neben dem Brüdenkondensat aus der Aufkonzentrierung der Nitrierendsäure (als SAC-Abwasser bezeichnet), das stets sauer ist vor allem das Abwasser aus der Wäsche mit Basen an, mit einem pH im Bereich von 7-10. Vor Abgabe in einen Vorfluter müssen aus diesen Abwässern neben allen toxischen, gentoxischen oder bakterostatisch wirkenden Stoffe aber auch alle anorganischen Salze, vor allem Nitrat und Nitrit soweit entfernt werden, dass die festgelegten Abgabeparameter erfüllt werden.

Vor allem das Abwasser aus der Wäsche des DNT mit Basen, das neben dem gelösten Produkt von bis zu 0,5%, entsprechend seiner Löslichkeit, die im Rohprodukt gelösten
phenolischen Nitroverbindungen, Nitrobenzoesäuren und anderen organischen Säuren und niedermolekularen Abbauprodukte enthält, bedarf eines hohen Aufwandes zur Entfernung der toxischen, krebserzeugenden, mutagenen und bakteriostatischen Nitroaromaten und phenolischen und sonstigen Verunreinigungen.
Eine große Anzahl der verschiedensten Verfahren wurden beschrieben, um diese toxischen Stoffe vor Abgabe aus dem alkalischen Waschwasser zu entfernen.

Neben einer rein mechanischen Abtrennung der Nitrokresole aus dem Abwasser einer Wäsche von DNT mit Basen, bevorzugt Soda, sind Verfahren zur Behandlung mit Aktivkohle oder lonenaustauscherharzen, Verfahren der Extraktion mit dem zu nitrierenden Aromaten, Behandlung mit Oxidationsmitteln, wie Ozon oder Fentonsreagenz, oder eine Behandlung bei hohen Temperaturen bis 300°C beschrieben worden, um neben den Nitrokörpern, vorwiegend dem Produkt, vor allen die phenolischen Nitrokörper, wie Di-und Trinitrophenole (Pikrinsäure), die Di- und Trinitrokresole (TNKs) aus dem Abwasser zu entfernen oder abzubauen, bevor diese Abwässer einer abschließenden Nachbehandlung in einer Kläranlage zugeführt werden.

In US 4 597 875 wird ein Verfahren beschrieben, bei dem die besonders toxischen Nitrokresole im Abwasser der Wäsche des DNT mit Soda durch Ausfällen entfernt werden. Durch Kreisführung der alkalischen Waschlauge werden die Nitrokresole bis zu einem Gehalt von ca. 1% Nitrokresol aufkonzentriert. Durch nachfolgendes Ansäuern dieser konzentrierten alkalischen Waschlauge auf einen pH unter 1 mit Schwefelsäure oder Abfallsäure werden die Nitrokresole ausgefällt, abgetrennt und durch Verbrennen entsorgt. Dieses Verfahren führt aber nur zu einer Reduzierung der toxischen Stoffe im Abwasser und nicht zu einer vollständigen Entfernung derselben aus dem Abwasser. Zusätzlich erfolgt eine starke Aufsalzung mit anorganischen Salzen, so dass kontinuierlich ein weiterhin mit Nitrokresolen belasteter Abwasserstrom, wenn auch in reduzierter Menge, ausgeschleust und weiter behandelt werden muss.

In US 4 482 769 wird ein weiteres Verfahren zur Reduktion des Gehaltes an Nitrokresolen im alkalischen Waschwasser aus der Wäsche des Roh-DNT beschrieben. Die im Roh-DNT gelösten Trinitrokresole werden bei einem pH um 6 in der alkalischen Waschlauge selektive ausgewaschen, während die Dinitrokresole im DNT verbleiben. Die Fracht an toxischen Nitrokresolen im zu behandelnde Abwasser aus der Wäsche des DNT wird dadurch deutlich reduziert. Die im DNT verbleibenden Dinitrokresole stören die Reduktion des DNT zu dem entsprechenden Amin nicht.

In allen diesen Verfahren wird nur eine teilweise Entfernung der im Wachwasser vorliegenden Nitroaromaten und Nitrokresole erreicht. Dieses Abwasser ist daher nach Abtrennung eines Teils der toxischen Verunreinigungen weiterhin stark belastet und bedarf, vor
Abgabe in einen Vorfluter, einer zusätzlichen aufwendigen Nachbehandlung.

Von R. Angst und M. Brem wird in Chimia 21,356-360 (1967) ein Verfahren zur Reinigung von Nitrokresole enthaltenden Abwässern aus einer Nitrierung von Toluol zu TNT durch behandeln mit Aktivkohle beschrieben. Eine mikrobiologische Reinigung der Abwässer in Kombination mit häuslichen Abwässern bewirkt, nach ihren Angaben, keine Veränderung bezüglich Toxizität und Farbe, während die Abwässer durch Behandlung mit Aktivkohle erfolgreich entfärbt werden. Nachteil der Behandlung mit Aktivkohle ist deren hoher Bedarf, da die mit Nitroaromat und Nitrokresolen beladene Aktivkohle wegen des Sprengstoffcharakters der TNKs und Nitroaromaten aus Sicherheitsgründen thermisch nicht regenerierbar ist, bzw. die Nitroaromaten durch Extraktion mit geeigneten Lösungsmitteln, nur zum Teil von der Aktivkohle wieder entfernt werden können.

In DE 1 221 994 und DE 1 222 904 wird ein Verfahren zur Entfernung der toxische Nitrokresole aus einem Waschwasser einer Wäsche von MNT und DNT beschrieben, bei dem die Nitrokresole mittels stark basischer Austauscherharze aus dem Abwasser bei einem pH größer 7 entfernt werden. Zur Regeneration des Austauscherharzes wird dasselbe mit Säuren und Aceton oder anderen geeigneten Lösungsmitteln behandelt. Das mit den Nitrokresolen beladene Aceton wird destillativ zurück gewonnen und die Nitrokresole werden anschließend vernichtet.

Ein weiteres, in zahlreichen Patenten beschriebenes Verfahren stellt die Extraktion der Abwässer aus der Wäsche und der Brüden aus der Aufkonzentrierung der Endsäure mit dem zu nitrierenden Aromaten dar.

In US 4 925 565 wird ein Verfahren zur Extraktion von Nitrophenol bzw. Nitrokresol enthaltenden Abwässern mit Toluol beschrieben. Bei einem pH von ca. 1 wird in einer dreistufigen Gegenstromextraktion das Abwasser fast vollständig von den Nitrophenolen befreit. In einer Destillationseinheit wird das Toluol zurückgewonnen und in die Extraktion zurückgeführt. Das im Kolonnensumpf anfallende Gemisch Nitroaromat/Nitrokresole wird verbrannt. Das Abwasser mit Spuren an Nitrophenolen wird, nach Strippung und vor Abgabe in einen Vorfluter einer Behandlung mit Aktivkohle unterzogen.

In US 3 742 072 wird ein Verfahren beschrieben, bei dem alle neutralen Nitrokörper, wie DNT und TNT aus einem "Mahon water" (Waschwasser aus der Abgaswäsche einer TNT-Anlage, speziell einer SAC-Anlage) durch Extraktion mit Toluol entfernt und das beladene

Toluol aus der Extraktion in die Nitrierung zurückgeführt wird.

In EP 1 003 710 wird ein Verfahren zur Extraktion des Abwassers aus der sauren Wäsche des Nitroaromaten, bevorzugt DNT, mit dem zu nitrierenden Aromaten (Toluol) beschrieben, um den in diesem sauren Waschwasser gelösten Nitroaromaten zu entfernen, bevor dieses Abwasser einer weiteren Behandlung, z.B. einer Aufkonzentrierung unterworfen wird. Das mit dem extrahierten Nitroaromaten beladene Extraktionsmittel wird in die Nitrierung zurückgeführt.

In US 6 506 948 wird ein Verfahren beschrieben, mit dem durch Extraktion der einzelnen Abwässer aus einer Anlage zur Herstellung von DNT (Brüdenkondensat, saures und am Schluss alkalisches Abwasser) mit Toluol im Gegenstrom die neutralen Nitrokörper, wie das DNT, selektiv abgetrennt und mit dem Toluol in die Nitrierung zurückgeführt werden. Das nur noch die Nitrokresole enthaltende Abwasser wird einem weiteren Abbau zur Entfernung der Nitrokresole unterworfen, bei dem die neutralen Nitrokörper nicht mehr stören.

In IT 912500 und CS 206 757 wird ein Verfahren beschrieben, mit dem nicht nur die neutralen Nitroaromaten, wie DNT und MNT aus der Wäsche des Nitroaromaten, sondern auch die Nitrokresole bei pH Werten kleiner 5 durch Extraktion mit dem zu nitrierenden Aromaten aus den vereinigten Waschwässern entfernt werden. Durch eine Rückextraktion aus dem Extraktionsmittel mit Alkali werden diese Nitrophenole, als hochkonzentrierte Lösung erhalten aus der, nach ansäuern, die Nitrophenole abgetrennt und einer Verbrennung zugeführt werden. Das Extraktionsmittel, der zu nitrierende Aromat, mit dem extrahierten DNT wird in die Nitrierung zurückgeführt.

In EP 1 493 730 und EP 1 496 043 wird ebenfalls ein Verfahren zur Teilreinigung eines vereinigten Abwassers aus der Herstellung von DNT beschrieben, bestehend aus dem Abwasser aus der sauren Wäsche und der alkalischen Wäsche des DNT und dem Brüdenkondensat aus der Aufkonzentrierung der MNT-Endsäure mit einem Gehalt an MNT/DNT von 0,7-13%. Der pH in diesem vereinigten Abwasser soll sich, durch eine geeignete Kombination der verschiedenen Abwasserströme, auf kleiner 5 einstellen. Durch die sich abscheidende MNT/DNT-Phase, aus dem Brüdenkondensat aus der SAC-Anlage, in einem Scheider werden bis zu 90% der im vereinigten Abwasser suspendierten Nitrokresole, Nitrobenzoesäuren extrahiert. Das sich abscheidende MNT/DNT- Gemisch mit einem Gehalt an Nitrokresolen und Nitrobenzoesäuren von z.B. 13,8 Gew.-% wird entweder in einer Verbrennung entsorgt oder, es werden mit einer dafür geeigneten Mischsäure (EP 1 493 730), die Nitrokresole oxidativ abgebaut. Das nach Abtrennung der MNT/DNT-Phase anfallende Abwasser kann noch weiter mit Toluol extrahiert werden, wobei das im vereinigten Abwasser noch gelöste MNT und DNT und ein weiterer Anteil der im
Abwasser noch gelösten Nitrokresole daraus entfernt werden. Dieser Toluolextrakt wird in die Nitrierung zurückgeführt. Das nach der Extraktion mit Toluol anfallende teilgereinigte Abwasser, das immer noch max 200 ppm an Nitrokresolen enthalten kann, kann nicht direkt abgegeben sondern muss vor Abgabe in einen Vorfluter weiter behandelt werden.

Alle diese Verfahren, Anreicherung der im Abwasser vorliegenden Schadstoffe und nachfolgende Entsorgung derselben, verbunden mit einer nur teilweisen Entfernung der Verunreinigungen aus den einzelnen oder vereinigten Abwässer aus der Wäsche des DNT, sind technisch aufwendig, benötigen zusätzliche Chemikalien und führen, durch das Sauerstellen der alkalischen Waschlauge und die Rückneutralisation auf einen pH um 7, zu einer zusätzlichen Aufsalzung der Abwässer.

Zur Vermeidung dieser technisch aufwendigen Anreicherungstechnologien wurden Verfahren entwickelt, die Schadstoffe, vor allem die Nitrokresole, ohne Zwischenanreicherung in den einzelnen oder vereinigten Abwässern, durch chemischen Abbau zu entsorgen.

In EP 0 962 446 wird ein Verfahren zur teilweisen Entfernung von im alkalischen Abwasser gelösten Nitroaromaten und Nitrokresolen durch Oxidation mit Salpetersäure beschrieben.
In Gegenwart von maximal 4 Gew.-% Salpetersäure und bis zu 4 Gew.-% Schwefelsäure bei einem pH kleiner 3 und nachfolgendem Erwärmen auf bis zu 180°C wird ein beträchtlicher Teil der Nitrokresole und des gelösten DNT in einem ursprünglich alkalischen Abwasser aus der Wäsche von DNT, abgebaut. Der TOC des alkalischen Abwassers wird um bis zu 50% abgesenkt. Das resultierende, teilgereinigte Abwasser muss einer weiteren Behandlung wie einer Behandlung mit Aktivkohle oder einer biologischen Behandlung zugeführt werden

In US 4 604 214 und US 5 356 539 wird ein Verfahren beschrieben, bei dem die im alkalischen Waschwasser einer Wäsche von DNT gelösten Nitrokresole durch Wasserstoffperoxid in Gegenwart von Eisen-II-Salzen (Fentons Reagenz) abgebaut werden. Durch mehrfache Kreisführung des alkalischen Waschwassers werden die Nitrokresole auf einen Gehalt von ca. 1 Gew.-% angereichert. Zur Behandlung wird dieses alkalische Waschwasser auf einen pH kleiner 4,5, bevorzugt 3, angesäuert, mit einer ausreichenden Menge an Wasserstoffperoxid (7-12 Mol/Mol TNK) und Eisensalzen versetzt und bei einer Temperatur von 70-90 °C abgebaut. Mehr als 90% der Nitrokresole und ca. 50% des gelösten DNT werden bei einer mittleren Verweilzeit von ca. 45 min im kontinuierlichen Reaktor abgebaut. Auch hier erfolgt nur eine Teilreinigung des alkalischen Waschwassers. Der Restgehalt an Nitrokresolen im so behandelten Abwasser von bis zu 200 ppm muss durch weitere Maßnahmen, z.B. Behandlung mit Aktivkohle, soweit reduziert werden, das eine Abgabe in einen Vorfluter nach den gültigen heutigen Standards möglich ist.

Neben der Oxidation mit Wasserstoffperoxid wurde auch gezielt Ozon als Oxidationsmittel zum Abbau von Nitrokresolen in einem alkalischen Waschwasser aus einer DNT-Anlage eingesetzt.

In EP 0 634 365 wird der Einsatz von Ozon, nach Abtrennung der neutralen Nitrokörper
aus dem alkalischen nitrophenolhaltigen Waschwasser einer Nitrobenzolanlage, beschrieben. In EP 0 953 546, Absatz 0007, wird ein Kombiverfahren, bestehend aus Adsorption an Klärschlamm zur teilweisen Entfernung von DNT, biologischer Vorbehandlung, Ozonisierung und biologischer Nachbehandlung eines nitrokresolhaltigen Abwassers aus einer DNT-Anlage beschrieben. Der mit DNT beladene Klärschlamm wird verbrannt.

Obwohl bei dem Einsatz von Oxidationsmitteln wie Salpetersäure, Wasserstoffperoxid oder Ozon keine aufwendige Zwischenkonzentrierung der toxischen Inhaltstoffe aus der alkalischen Wäsche eines Roh-DNT, wie den Nitroaromaten und Nitrokresolen, vor deren Beseitigung nötig ist, ist der zusätzliche Bedarf an Oxidationsmitteln in Verbindung mit aufwendigen biologischen Zwischenreinigungs- und Endreinigungsschritten kompliziert, aufwendig und teuer.

In EP 0 581 229 wird eine Nassoxidation von alkalischem Abwasser, das noch alle Nitrokörper und Nitrokresole aus einer Wäsche von DNT enthält, in Gegenwart von zusätzlichem Oxidationsmittel wie Sauerstoff oder Luft unterhalb des überkritischen Temperaturbereichs für Wasser bei 325-370°C aber bei überkritischem Druck von 220-325 bar beschrieben, bei dem das Oxidationsmittel vollständig im Abwasser gelöst vorliegt und die im Abwasser vorhandenen und durch die Oxidation gebildeten Salze (Karbonate) gelöst bleiben und nicht durch Verdampfungsreaktionen abgeschieden werden (und damit die Thermolyse behindern). Die für den oxidativen Abbau benötigten Verweilzeiten sind kurz. Das Abwasser nach einer Thermolyse in Gegenwart von Oxidationsmittel ist noch gelb gefärbt und muss, vor Abgabe in einen Vorfluter, noch nachbehandelt werden.

Zur Vermeidung von Zwischenreinigungsschritten und einem zusätzlichen Bedarf an teuren Hilfsmitteln, wie Säuren, Basen und Oxidationsmitteln. bei der Entfernung biologisch nicht abbaubarer Nitrokörper und Nitrophenole in einem Abwasser aus der Herstellung von DNT wurden zusätzlich Verfahren beschrieben mit denen diese Nachteile, vor allem der zusätzliche Bedarf von Hilfsmitteln, vermieden werden kann.

In EP 0 005 203 wird ein Thermolyseverfahren zur direkten Aufbereitung von Nitrokresole enthaltenden alkalischen Abwasser aus einer Nitrieranlage zur Herstellung von DNT beschrieben, für das keine zusätzlichen Chemikalien noch andere Zwischenschritte benötigt werden. Das Abwasser aus der alkalischen Waschstufe einer DNT-Anlage, frei von den
neutralen Nitroaromaten wie DNT; wird, unter Luft und Sauerstoffausschluss, bei 290-300°C einem Druck von ca. 100-110 bar, direkt 15 min. behandelt. Nach Abkühlen auf Raumtemperatur sind alle Natriumsalze der Nitrokresole zu CO₂, CO Stickstoff und anderen kurzkettigen Abbauprodukten der Nitrokresole abgebaut.

In EP 0 953 546 wird ein Verfahren beschrieben, das es sogar erlaubt die Thermolyse in Gegenwart der neutralen Nitrokörper wie DNT im Abwasser einer alkalischen Waschstufe aus einer DNT-Anlage durchzuführen. Die aufwendige Entfernung der keine Hydroxygruppe aufweisenden Nitroaromaten wie die Nitrotoluole oder Dinitrotoluole, durch Extraktion oder Wasserdampfstrippung vor Einspeisung in die Thermolyse entfällt.

In EP 1 132 347 wird eine Thermolyse im überkritischen Bereich für ein vorkonzentriertes alkalisches Waschwasser aus der Wäsche von Nitrobenzol beschrieben. Durch die Vorkonzentrierung wird die Hauptmenge der im Waschwasser gelösten, mit Wasserdampf flüchtigen Nitroaromaten, im vorliegenden Fall Nitrobenzol, aus dem Waschwasser abgetrennt. Nur die als Salze gelösten Nitrophenole bleiben zurück und werden bei 500-600°C und 25 MPa (250 bar) vollständig abgebaut. Das Destillat aus der Vorkonzentrierung wird in die Wäsche zurückgeführt. Das Abwasser aus der Thermolyse enthält nur noch geringe Mengen an organischen Verunreinigungen und kann in einer Kläranlage nachbehandelt werden.

Die alkalischen Abwässer aus einer Wäsche des DNT in der alkalischen Waschstufe sind, nach Verlassen der Thermolyse, im allgemeinen frei von Nitrokresolen und DNT, sind aber zum Teil tief gefärbt durch kolloidal abgeschiedenen Kohlenstoff, und enthalten, neben niedermolekularen Abbauprodukten unbekannter Konstitution aus DNT und den Nitrokresolen, Ammoniak, neu gebildetes Natriumkarbonat und Spuren von gelöstem CO. Der TOC wurde im Vergleich zum unbehandelten Wasser um ca. 20% und der CSB um etwa 30-35 % in der Thermolyse reduziert. Überraschend ist, dass Mononitroaromaten, wie MNT, deutlich langsamer als die Di- und Trinitroaromaten, und die Nitrobenzoesäuren fast gar nicht unter den Bedingungen der Thermolyse abgebaut werden und unverändert im Abwasser verbleiben. Dieses Abwasser aus der Thermolyse muss deshalb, vor Abgabe in einen Vorfluter unter Einhaltung der jeweils gültigen Vorschriften bezüglich Farbe, CSB, TOC und Toxizität noch nachbehandelt werden z. B. in einer Kläranlage.

Die Behandlung von biologisch nicht oder nur schwer abbaubaren Abwässern, die neutrale Nitroaromaten wie DNT und noch toxischere Nitrophenole und Nitrokresole enthalten in einer Thermolyse ist sehr effektiv, aber technisch aufwendig und teuer.

Alle vorstehend beschriebenen Verfahren, Einrichtungen und Maßnahmen zur Aufbereitung des Abwassers aus der Herstellung von DNT, besonders des mit den toxischen und schwer oder nicht abbaubaren Nitrokresolen belasteten Abwassers aus der Wäsche von DNT mit Alkalien, umfasst zwei Stufen, nämlich
a) der Abtrennung des im Wasser gelösten DNT und der toxischen Nitrokresole so weit wie möglich,
b) die Nachbehandlung des nach a) vorgereinigten Abwassers z.B. in einer Kläranlage oder durch Behandlung mit Adsorbentien, vor der endgültigen Abgabe in einen Vorfluter.

Alle diese Verfahren und Einrichtungen zur Behandlung dieser mit Nitrokörpern belasteten Abwässer, vor allem die Vorbehandlung nach a), sind technisch aufwendig und teuer in Investition und Betrieb, benötigen zusätzliche Chemikalien und führen teilweise zu einer zusätzlichen Belastung mit anorganischen Salzen, die durch das Ansäuern und die anschließende Neutralisation in das zu behandelnde Abwasser eingebracht werden. Zusätzlich führt das Versetzen der alkalischen Abwässer aus der DNT-Wäsche mit Säuren zwecks Abtrennung der Nitrokresole bei pH Werten kleiner 5 zu der Entwicklung beträchtlicher Mengen von CO₂, die durch ihre lästige Schaumbildung verbunden mit der beträchtlichen Abgasbelastung zu Problemen führt.

Zusätzlich sind, um die Belastung des Abwassers aus der Wäsche des Roh-DNT mit den toxischen Nitrokresolen zu reduzieren, auch Verfahren beschrieben worden, bei denen ein Teil Nitrokresole und Nitrophenole im Produkt (DNT) verbleibt und erst nach der Hydrierung des DNT zu Diaminotoluol (TDA) mit den "heavy ends" zusammen abgetrennt und entsorgt wird.

In US 2 976 320 und US 4 482 769 werden Verfahren beschrieben bei denen nur ein Teil der im Roh-DNT vorliegenden Nitrokresole und Nitrophenole im DNT verbleiben (max 500 ppm, bevorzugt nicht mehr als 200 ppm). Damit resultiert ein Abwasser bei der Wäsche, in dem weiterhin die Hauptmenge der im Roh-DNT vorliegenden "Nitrophenole" enthalten ist. Dieses Abwasser muss weiterhin nach dem Stand der Technik vor Abgabe in einen Vorfluter behandelt werden.

In CA 1 034 603 wird ein Verfahren beschrieben bei dem das Roh-DNT nur mit Wasser gewaschen wird, um die Mineralsäuren, die die Hydrierung negativ beeinflussen, so vollständig wie möglich zu entfernen (mindestens 99.8%). Ein Teil der im Roh-DNT gelösten Nitrokresole verbleibt dabei im DNT und wird auf der TDA-Stufe abgetrennt. Von den ursprünglich im Roh-DNT vorhandenen Nitrokresolen gelangen noch bis zu fast 40% in das

Abwasser. Außerdem sind in diesem Abwasser die gesamten im Roh-DNT ursprünglich vorhandenen Mineralsäuren gelöst. Diese Waschwässer müssen, vor allem das Nitrat, neben den restlichen Nitrokresolen vor Abgabe desselben in den Vorfluter aufwendig nach den Stand der Technik entfernt werden.

In US 4 224 249 wird ein Verfahren zur Hydrierung von mit Wasser nur unvollständig gewaschenem DNT mit einem Rest an Säure (bis zu 6000 ppm als Schwefelsäure bzw. 7700 ppm als Salpetersäure) mit Raney-Nickel zu Diaminotoluol (TDA) beschrieben. Wie aber
die Beispiele zeigen wird bei einer Restacidität von ca. 6000 ppm im gewaschenen DNT in Gegenwart von ca. 800 ppm an Nitrokresolen die Aktivität des Katalysators schon nach kurzer Zeit auf null reduziert. Auch bei der nicht näher beschriebenen Wäsche gelangen ca. ein Drittel der im Roh-DNT ursprünglich vorhandenen "Nitrophenole" in das Waschwasser und müssen, entsprechend dem vorstehend beschriebenen Stand der Technik vor Abgabe desselben in den Vorfluter aufwendig entfernt werden.

Aufgabe der Erfindung ist es, ein Verfahren zur Wäsche des bei der Nitrierung von Toluol nach Abtrennung der Nitriersäure anfallenden, Dinitrotoluol enthaltenden Rohgemischs bereitzustellen, das es gestattet, das Abwasser aus dieser Wäsche direkt und ohne aufwendige Zwischenbehandlung zur Abtrennung bzw. Zerstörung der Nitrokresole und Nitrobenzoesäuren in die biologische Behandlungsstufe einer Kläranlage abzugeben. Das in der biologischen Stufe behandelte Abwasser soll, unter Einhaltung aller einschlägigen gesetzlichen Auflagen, z.B. der Vorgaben der Abwasserabgabeverordnung der Bundesrepublik Deutschland, in einen Vorfluter eingeleitet werden können. Insbesondere ist es Aufgabe der Erfindung, ein Verfahren zur Wäsche des DNT-haltigen Rohgemischs bereitzustellen, bei dem die in dem DNT-haltigen Rohgemisch vorliegenden Nitrobenzoesäuren (Mono- und Dinitrobenzoesäuren) nicht in das Abwasser der Wäsche gelangen. Weiterhin ist es Aufgabe der Erfindung, ein solches Verfahren bereitzustellen, bei dem der Gehalt an Salpetersäure und Schwefelsäure in dem DNT-haltigen Rohgemisch minimiert und gleichzeitig die Belastung des Abwassers mit Sulfat und Nitrat minimiert wird. Weiterhin ist es Aufgabe der Erfindung, ein solches Verfahren bereitzustellen, bei dem nach der Wäsche ein "technical grade DNT" erhalten wird, in dem die aus dem oxidativen Abbau der Nitrokresole auf der DNT-Stufe der Nitrierung auffallenden, bekannten Abbauprodukte und potentiellen Katalysatorgriffe, die bei der katalytischen Reduktion des DNT mit Wasserstoff stören können, wie Blausäure (HCN), Distickstoffoxid (N₂O), Stickoxide (NOₓ) und Kohlenmonoxid (CO) minimiert werden.
Gelöst wird die Aufgabe durch ein Verfahren zur Wäsche eines bei der Nitrierung von Toluol nach Abtrennung der Nitriersäure anfallenden Rohgemischs enthaltend Dinitrotoluol, Salpetersäure, Stickstoffoxide und Schwefelsäure umfassend zwei Waschschritte (WS-I) und (WS-II), wobei
i) in einem ersten, im Gegenstrom durchgeführten Waschschritt (WS-I) das Rohgemisch in einer mindestens zwei Extraktionsstufen umfassenden Wäsche mit einer Salpetersäure, Stickstoffoxide und Schwefelsäure enthaltenden Waschsäure I extrahiert wird, wobei der letzten Extraktionsstufe (WS-I-n) des ersten Waschschrittes (WS-I) Frischwasser oder eine Waschsäure mit einem Gesamtsäuregehalt von 0,2 bis 1,5 Gew.-% zugeführt wird und die aus der ersten Extraktionsstufe (WS-I-1) des ersten Waschschrittes (WS-I) abgeführte Waschsäure einen Gesamtsäuregehalt von 20 bis 40 Gew.-% aufweist, wobei ein vorgewaschenes, Dinitrotoluol enthaltendes Rohgemisch erhalten wird,
ii) in einem zweiten Waschschritt (WS-II) das vorgewaschene Rohgemisch in einer mindestens eine Extraktionsstufe umfassenden Wäsche mit einer Waschsäure II extrahiert wird, wobei der letzten Extraktionsstufe (WS-II-m) des zweiten Waschschrittes (WS-II) Frischwasser zugeführt wird und die aus der ersten Extraktionsstufe (WS-II-1) des zweiten Waschschrittes (WS-II) abgeführte Waschsäure einen pH-Wert von kleiner oder gleich 4 aufweist, wobei ein von Salpetersäure und Schwefelsäure im wesentlichen freies Dinitrotoluol enthaltendes Gemisch erhalten wird.

Das Rohgemisch kann darüber hinaus Spuren von Blausäure, Distickstoff und Kohlenmonoxid enthalten.
Überraschender Weise wurde gefunden, dass bei der erfindungsgemäßen sauren Wäsche umfassend einen ersten Waschschritt (WS-I) und einen zweiten Waschschritt (WS-II) in dem zweiten Waschschritt ein Abwasser erhalten wird, das ohne Zwischenbehandlung zur Abtrennung von Nitrokresolen (Dinitro- und Trinitrokresolen) und der Nitrobenzoesäuren (Mono- und Dinitrobenzoesäure) sowie von oxidativen Abbauprodukte der Dinitrokresole in die biologische Behandlungsstufe einer Kläranlage eingebracht werden kann. Das nach Verlassen der biologischen Behandlungsstufe erhaltene Abwasser kann direkt und unter Einhaltung der in einschlägigen Abwasserverordnungen, beispielsweise der Bundesrepublik Deutschland, vorgegebenen Grenzwerte in einen Vorfluter abgegeben werden. Insbesondere war überraschend, dass bei Einhaltung des pH-Wertes in der ersten Extraktionsstufe des zweiten Waschschritts sich nahezu die Gesamtmenge der Nitrobenzoesäuren in der DNT enthaltenden organischen Phase wiederfindet und nicht in das Abwasser übergeht.
Dabei wird auch im Vergleich zum Stand der Technik, wie er beispielsweise in US 2 976 320, US 4 482 769 und CA 1 034 603 beschrieben ist, die Abwasserbelastung mit Sulfat und vor allem mit Nitrat, das nur aufwendig aus dem Abwasser entfernt werden kann, wesentlich reduziert.
Das erfindungsgemäße Verfahren stellt im Sinne der EU-Richtlinie zur integrierten Verminderung und Vermeidung von Umweltverschmutzung (IVU-RL) eine Weiterentwicklung der besten verfügbaren Techniken (BVT), wie sie in den BREFs (BVT-Referenz-Dokument) konkretisiert werden, dar. Mit dem erfindungsgemäßen Verfahren kann nicht nur die gesamte Abwassermenge aus einer DNT-Anlage deutlich reduziert werden, sondern es können auch die umfangreichen und technisch aufwendigen Maßnahmen zur Entfernung oder teilweisen Entfernung der Nitrokresole, Nitrobenzoesäuren und sonstigen Abbauprodukte aus dem Abwasser entfallen.

Dabei werden im ersten Waschschritt (WS I) in einer mindestens zweistufigen Extraktion mit einer Waschsäure als Extraktionsmittel die in dem DNT enthaltenden Rohgemisch gelösten Mineralsäuren Schwefelsäure und Salpetersäure sowie Stickstoffoxide abgetrennt.

Der erste Waschschritt wird mindestens zweistufig und im Gegenstrom durchgeführt. Die mindestens zweistufige Gegenstromextraktion kann prinzipiell wie in EP 0 279 312, EP 0 736 514 oder EP 1 780 195 beschrieben erfolgen.
Im Gegenstrom wird der zweite Waschschritt im Allgemeinen dergestalt durchgeführt, dass
I) in der ersten Extraktionsstufe (WS-I-1) des ersten Waschschritts (WS-I) das Rohgemisch enthaltend Dinitrotoluol, Salpetersäure, Stickstoffoxide und Schwefelsäure in einen
   ersten Mischer (M-I-1) zusammen mit im Kreis geführter Waschsäure (WSR-I-1) aus einem zu dieser ersten Extraktionsstufe gehörenden ersten Phasentrennapparat (S-I-1) und überschüssiger Waschsäure (WSR-I-2) aus der nachfolgenden Extraktionsstufe (WS-I-2) eingespeist wird, die in dem Mischer gebildete Waschemulsion in dem ersten Phasentrennapparat (S-I-1) in die Waschsäure (WSR-I-1) und einmal gewaschenes Rohgemisch (DNT-I-1) aufgetrennt, und
II) das einmal gewaschene Rohgemisch (DNT-I-1) in der nachfolgenden Extraktionsstufe (WS-I-2) des ersten Waschschritts (WS-I) in einen zu dieser Extraktionsstufe gehörenden zweiten Mischer (M-I-2) zusammen mit der im Kreis geführten Waschsäure (WSR-I-2) aus einem zu dieser Extraktionsstufe gehörenden zweiten Phasentrennapparat (S-I-2) und, wenn der erste Waschschritt (WS-I) mehr als 2 Extraktionsstufen umfasst, mit überschüssiger Waschsäure (WSR-I-3) aus der nachfolgenden Extraktionsstufe (WS-I-3) oder, wenn der erste Waschschritt genau 2 Extraktionsstufen umfasst, mit Frischwasser oder Waschsäure eingespeist wird, wobei, wenn der erste Waschschritt mehr als 2 Extraktionsstufen umfasst, sich weitere Extraktionsschritte entsprechend den Schritten I) und II) anschließen können.

Die Gegenstromextraktion kann bis zu n Extraktionsstufen aufweisen. Dabei wird das Extraktionsmittel der letzten der n Extraktionsstufen (WS-I-n) des ersten Waschschritts (WS-I) zugeführt. Das zugeführte Extraktionsmittel, das dieser letzten Extraktionsstufe zugeführt wird, kann Frischwasser oder eine Waschsäure mit einem bestimmten Gesamtsäuregehalt sein. In einer Ausführungsform der Erfindung ist das zugeführte Extraktionsmittel Wasser. In einer weiteren Ausführungsform ist das zugeführte Extraktionsmittel eine Waschsäure mit einem Gesamtsäuregehalt im Bereich von 0,2 bis 1,5 Gew.-%. Beispielsweise kann diese Waschsäure das Brüdenkondensat aus der Aufkonzentrierung der Waschsäure, die im ersten Extraktionsschritt (WS-I-1) der ersten Waschstufe (WS-I) gewonnen wird, sein. Diese enthält im Allgemeinen bis zu 1,0 Gew.-% Salpetersäure und bis zu 0,3 Gew.-% Schwefelsäure.

Die in dem ersten Waschschritt in der ersten Extraktionsstufe anfallende Waschsäure weist im Allgemeinen einen Gesamtsäuregehalt von 20 bis 40 Gew.-% auf. Dieser Gesamtsäuregehalt ist die Summe aus Salpetersäure, Schwefelsäure und Stickoxiden, wobei Stickoxide als Salpetrige Säure HNO₂ berechnet werden.

Die in dem ersten Waschschritt auf der ersten Extraktionsstufe anfallende Waschsäure kann direkt oder nach Aufkonzentrierung in die Nitrierung von Toluol zurückgeführt werden. Sie kann auch in die Aufkonzentrierung der Endsäure aus der ersten Stufe der Toluol-Nitrierung (Mononitrotoluol (MNT)-Stufe) eingebracht werden. In diesem ersten Waschschritt (WS-I) wird im Wesentlichen die Belastung des im zweiten Waschschritt anfallenden Abwassers mit Sulfat und Nitrat minimiert. Letzteres kann nur durch eine aufwendige Denitrifizierungsstufe aus dem Abwasser entfernt werden.

Das nach dem ersten Waschschritt anfallende vorgewaschene, DNT enthaltende Rohgemisch weist im Allgemeinen einen Gehalt an Sulfat von maximal 300 ppm, bevorzugt von maximal 100 ppm und besonders bevorzugt von maximal 50 ppm und einem Gehalt an Salpetersäure und Stickoxiden von maximal 5000 ppm, bevorzugt von maximal 3000 ppm und besonders bevorzugt von maximal 1000 ppm auf.

Im zweiten Waschschritt (WS II) werden mit einer mindestens einstufigen Extraktion mit einer Waschsäure, die nur noch einen geringen Gehalt an Gesamtsäure aufweist (im Wesentlichen Salpetersäure, Stickstoffoxide und Schwefelsäure) aus dem vorgewaschenen, DNT enthaltenen Rohgemisch die noch verbliebenen Reste an Salpetersäure, Stickstoffoxiden und Schwefelsäure derart ausgewaschen, dass der Gehalt an Mineralsäuren und Stickstoffoxiden im dem DNT enthaltenden Rohgemisch auf ein Minimum reduziert wird.

Vorzugsweise wird der zweite Waschschritt mindestens zweistufig und im Gegenstrom durchgeführt. Die Gegenstromextraktion kann bis zu m Extraktionsstufen aufweisen. Dabei wird das Extraktionsmittel der letzten der m Extraktionsstufen (WS-II-m) des zweiten Waschschritts (WS-II) zugeführt. Das zugeführte Extraktionsmittel ist im Allgemeinen Frischwasser.

Im Gegenstrom wird der zweite Waschschritt im Allgemeinen dergestalt durchgeführt, dass
I) in der ersten Extraktionsstufe (WS-II-1) des zweiten Waschschritts (WS-II) das vorgewaschene Rohgemisch enthaltend Dinitrotoluol in einen zu dieser Extraktionsstufe gehörenden ersten Mischer (M-II-1) zusammen mit im Kreis geführter Waschsäure (WSR-II-1) aus einem zu dieser Extraktionsstufe gehörenden ersten Phasentrennapparat (S-II-1) und überschüssiger Waschsäure (WSR-II-2) aus der nachfolgenden Extraktionsstufe (WS-II-2) eingespeist wird, die in dem ersten Mischer gebildete Waschemulsion in dem ersten Phasentrennapparat (S-II-1) in die Waschsäure (WSR-II-1) und einmal gewaschenes Rohgemisch (DNT-II-1) aufgetrennt, und
II) das einmal gewaschene Rohgemisch (DNT-II-1) in der nachfolgenden Extraktionsstufe (WS-II-2) des zweiten Waschschritts (WS-II) in einen zu dieser Extraktionsstufe gehörenden zweiten Mischer (M-II-2) zusammen mit der im Kreis geführten Waschsäure (WSR-II-2) aus einem zu dieser zweiten Extraktionsstufe gehörenden zweiten Phasentrennapparat (S-II-2) und, wenn der zweite Waschschritt (WS-II) mehr als 2 Extraktionsstufen
   umfasst, mit überschüssiger Waschsäure (WSR-I-3) aus der nachfolgenden Extraktionsstufe (WS-I-3) oder, wenn der Waschschritt genau 2 Extraktionsstufen umfasst, mit Frischwasser eingespeist wird,
   wobei, wenn der zweite Waschschritt mehr als 2 Extraktionsstufen umfasst, sich weitere Extraktionsschritte entsprechend den Schritten I) und II) anschließen können.

Dabei wird der zweite Waschschritt derart durchgeführt, dass im Wesentlichen die Gesamtmenge an Nitrokresolen und Nitrophenolen und vorzugsweise bis zu 95 Gew.-% der in dem ursprünglich in dem Rohgemisch vorhandenen Nitrobenzoesäuren in dem Rohgemisch verbleiben und nicht mit der Waschsäure ausgetragen werden. Dies wird erfindungsgemäß dadurch erreicht, dass die aus der ersten Extraktionsstufe (WS-II-1) des zweiten Waschschrittes (WS-II) abgeführte Waschsäure einen pH-Wert von kleiner oder gleich 4 aufweist. In der als Abwasser ausgeschleusten, niedrig konzentrierten Waschsäure mit einem pH-Wert von 4 oder kleiner ist der Gehalt an Nitrokresolen und Nitrophenolen im Allgemeinen insgesamt kleiner als 5 ppm und der Gehalt an Nitrobenzoesäuren im Allgemeinen kleiner 50 ppm. Dieses Abwasser, das nur noch das gewaschene DNT bis zur Sättigungsgrenze gelöst enthält, kann ohne die bisher übliche und notwendige Vorbehandlung zur Entfernung der Nitrokresole und der Nitrobenzoesäuren sowie toxischer oxidativer Abbauprodukte, direkt oder nach Rückgewinnung von gelöstem DNT, einer Kläranlage zugeführt werden.

Vorzugsweise erfolgt in jedem der beiden Waschschritten (WS-I) und (WS-II) die Wäsche in mindestens zwei Stufen im Gegenstrom. In einer Ausführungsform umfasst der erste Waschschritt (WS-I) 2 bis 4 Extraktionsstufen (n = 2 - 4). In einer weiteren Ausführungsform umfasst der zweite Waschschritt (WS-II) 2 bis 4 Extraktionsstufen (m = 2 - 4). Es können auch mehr Extraktionsstufen, beispielsweise 5 oder 6, pro Waschschritt vorgesehen werden. In einer Ausführungsform umfasst jeder Waschschritt genau 2 Extraktionsstufen.

Die in der letzten Extraktionsstufe eines jeden Waschschrittes zuzugebende Extraktionsmittel- bzw. Frischwassermenge hängt dabei davon ab, welcher Gesamtsäuregehalt (WS-I) bzw. welcher pH-Wert (WS-II) in der jeweils ersten Extraktionsstufe des jeweiligen Waschschrittes (WS-I) bzw. (WS-II) vorliegen soll.

Das Volumenverhältnis von DNT enthaltendem Rohgemisch (organischer Phase) einerseits zu Waschsäure (wässriger Phase) andererseits, die in jedem Waschapparat in direktem Kontakt miteinander stehen, wird in beiden Waschschritten im Allgemeinen von 1 : 4 bis 10 : 1, bevorzugt von 1 : 3 bis 5 : 1 und besonders bevorzugt von 1 : 3 bis 2 : 1 gewählt. Je nach Phasenverhältnis und Energieeintrag bei der Dispergierung kann das Gemisch als Öl in Wasser- (O/W-Emulsion) oder als Wasser in Öl-Emulsion (W/O-Emulsion) vorliegen.
Diese Phasenverhältnisse können durch Zugabe der entsprechenden Extraktionsmittelmenge zu der letzten Extraktionsstufe, bevorzugt aber, bei definierter Extraktionsmittelmenge, durch Kreisführung der Waschsäure nach Phasentrennung eingestellt werden, wobei nur der Überschuss (entsprechend der in der letzten Extraktionsstufe frisch zugeführten Extraktionsmittelmenge) in die vorhergehende Extraktionsstufe eingespeist bzw. ausgeschleust wird.

Der pH-Wert der Waschsäure, die aus der ersten Extraktionsstufe (WS-II-1) des ersten Waschschrittes (WS II) abgezogen wird, liegt im Allgemeinen im Bereich von 0 bis 3, bevorzugt von 0,5 bis 2 und besonders bevorzugt von 0,8 bis 1,2. Der gewünschte, optimale pH-Wert in der ersten Extraktionsstufe des zweiten Waschschritts kann durch den Restgehalt an Salpetersäure des aus dem ersten Waschschritt stammenden, vorgewaschenen Rohgemischs und/oder über die in der letzten Extraktionsstufe des zweiten Waschschritts zugesetzte Frischwassermenge eingestellt werden. Alternativ dazu kann der gewünschte pH-Wert auch durch Zugabe einer Mineralsäure, beispielsweise der Waschsäure aus dem ersten Waschschritt, oder von Schwefelsäure oder bevorzugt Salpetersäure eingestellt werden.

Das Verhältnis von DNT zu Frischwasser, das in der letzten Extraktionsstufe des zweiten Waschschrittes zugesetzt wird, kann, auf Volumenbasis, von 1 : 2 bis 15 : 1, entsprechend 1,5 m³ bis 0,05 m³ Frischwasser pro t DNT, bevorzugt von 1 : 1 und 7:1, entsprechend 0,75 m³ bis 0,107 m³ Frischwasser pro t DNT, und besonders bevorzugt von 1 : 1 bis 2 : 1, entsprechend 0,75 m³ bis 0,38 m³ Frischwasser pro t DNT, variiert werden. In beiden Waschschritten wird bei einer Temperatur oberhalb des Schmelzpunktes von DNT, im Allgemeinen bei 60 - 75 °C, gearbeitet.

Als Waschapparate für beide Waschschritte können beispielsweise Mixer-Settler-Apparate oder gerührte mehrstufige oder gepulste Packungs- und Siebbodenkolonnen, aber auch
statische Mischer in Verbindung mit geeigneten Scheideapparaten eingesetzt werden. Für die Trennung der Waschdispersion aus zu waschendem DNT-haltigen Rohgemisch und Waschsäure sind sowohl statische Abscheider wie dynamische Abscheider (Zentrifugalseparatoren) geeignet.

Die aus der ersten Extraktionsstufe (WS-II-1) des zweiten Waschschritts (WS-II) ausgeschleuste Waschsäure mit einem pH-Wert von kleiner oder gleich 4, bevorzugt 0 bis 3, besonders bevorzugt 0,5 bis 2 und insbesondere 0,8 bis 1,2 weist im Allgemeinen einen Gehalt an Sulfat von maximal 100 ppm, einem Gehalt an Salpetersäure von maximal 2000 ppm und einem Gehalt an HNO₂ von maximal 50 ppm auf. Daneben enthält sie, entsprechend der Sättigungsgrenze bei der vorgegebenen Temperatur, beispielsweise 800 bis 1000 ppm isomere Dinitrotoluole. Der Gehalt an Nitrokresolen und Nitrophenolen ist im Allgemeinen kleiner als 1 ppm, der Gehalt an Nitobenzoesäuren ist im Allgemeinen kleiner als 50 ppm ist.

Der TOC-Gehalt (total organic carbon) dieses Abwassers beträgt beispielsweise nur noch ca.1100 mg/l - gegenüber ca. 3000 mg/l in einem Abwasser aus der alkalischen DNT-Wäsche mit Soda. Der CSB-Gehalt (chemischer Sauerstoffbedarf) dieses Abwassers beträgt beispielsweise nur noch ca. 3000 mg O/I - gegenüber ca. 6000 mg O/I in einem Abwasser aus der alkalischen DNT-Wäsche mit Soda. Dieses Abwasser kann direkt oder nach Rückgewinnung des gelösten DNT durch Extraktion mit Toluol, ohne weitere Vorbehandlung wie Thermolyse oder Behandlung mit Oxidationsmitteln, der biologischen Behandlungsstufe einer Kläranlage zugeführt werden und erfüllt danach im Allgemeinen alle Anforderungen der Abwasserverordnung der Bundesrepublik Deutschland bezüglich Toxizität einschließlich Gentoxizität, bestimmt nach DIN EN ISO 38415 T6 (Fisch), 11348-2 (Leuchtbakterien), 38412 L30 (Daphnien), 38412 L33 (Algen) und DIN EN ISO 9888 (Umu Test, Gentoxizität).

Im Allgemeinen wird die aus der ersten Extraktionsstufe (WS-II-1) des zweiten Waschschritts (WS-II) abgeführte Waschsäure neutralisiert und kann, gegebenenfalls nach Abtrennung von darin gelöstem Dinitrotoluol durch Extraktion und Abtrennung des Extraktionsmittels, ohne weitere Vorbehandlung der biologischen Behandlungsstufe einer Kläranlage zugeführt werden.

Aus dem zweiten Waschschritt (WS-II) wird aus der letzten Extraktionsstufe ein DNT abgezogen, welches eine Restacidität von im Allgemeinen maximal 300 ppm (als Schwefelsäure bestimmt) und einen pH-Wert von 2-4 aufweist und im Allgemeinen maximal 800 ppm an Nitrokresolen und Nitrophenolen, maximal 600 ppm an Nitrobenzoesäuren (Dinitro- und Mononitrobenzoesäuren) sowie einem Restgehalt von im Allgemeinen maximal 300 ppm, bevorzugt maximal 200 ppm und besonders bevorzugt maximal 100 ppm

Salpetersäure (NOₓ, als Salpetersäure bestimmt),maximal 50 ppm, bevorzugt maximal 10 ppm und besonders bevorzugt maximal 1 ppm Blausäure, maximal 200 ppm, bevorzugt maximal 50 ppm und besonders bevorzugt maximal 25 ppm Distickstoffmonoxid, maximal 400 ppm, bevorzugt maximal 200 ppm und besonders bevorzugt maximal 50 ppm CO, bevorzugt und maximal 3 ppm Sulfat aufweist. Dieses DNT und die darin gelösten Nitrokresole, Nitrophenole und Spuren von NOₓ, Blausäure, Distickstoffmonoxid und CO, Nitrobenzoesäuren können entsprechend dem Stand der Technik vollständig und problemlos zu Toluylendiamin (TDA) und den entsprechenden Aminokresolen, Aminophenolen und Aminobenzoesäuren (Diamino- und Monoaminobenzoesäuren) hydriert werden. Die Aminokresole, Aminophenole und Aminobenzoesäuren verbleiben bei der TDA-Destillation im Destillationsrückstand und werden zusammen mit diesem entsorgt.

Ein weiterer Vorteil ist die höhere thermische Stabilität des erfindungsgemäß einer sauren Wäsche unterworfenen DNT. So wird die Temperatur, bei der die thermische Zersetzung beginnt, bei einem nur sauer gewaschenen DNT, welches beispielsweise 620 ppm an Nitrophenolen und Nitrokresolen und 460 ppm an Nitrobenzoesäuren enthält, im Vergleich zu einem DNT, das nach dem Stand der Technik in Gegenwart von Soda gewaschen wurde und beispielsweise weniger als 20 ppm an Nitrophenolen und Nitrokresolen enthält, um ca. 20 °C angehoben.

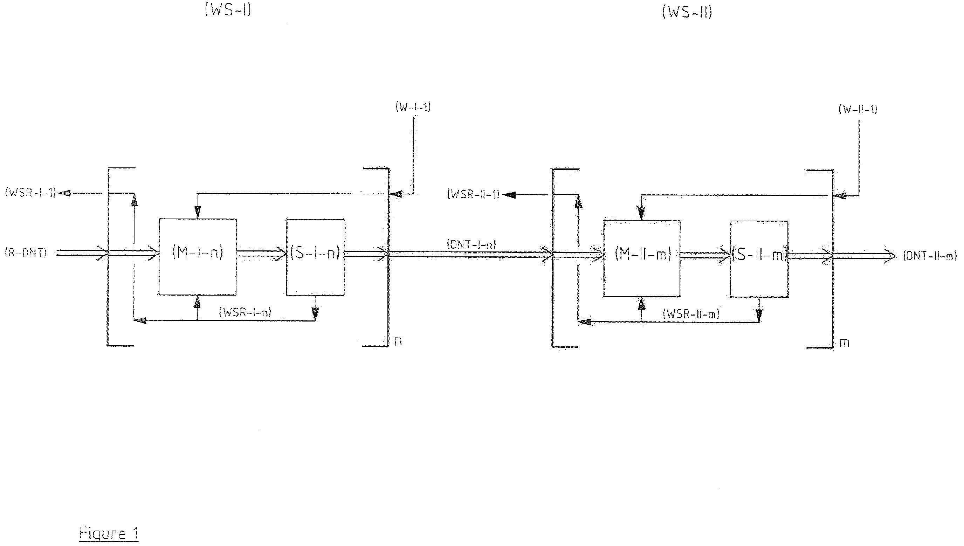

Figur 1 zeigt eine schematische Darstellung einer Wäsche von Roh-DNT nach dem erfindungsgemäßen Verfahren gemäß einer bevorzugten Ausführungsform der Erfindung.

Das rohe DNT (R-DNT) aus der Nitrierung nach Abtrennung der DNT-Endsäure wird im Waschschritt I (WS-I), in der ersten Extraktionsstufe (WS-I-1) (n=1) zusammen mit der im Kreis geführten Waschsäure (WSR-I-1) aus dem Phasentrennapparat (S-I-1) zusammen mit der überschüssigen Waschsäure (WSR-I-2) aus der nachfolgenden Extraktionsstufe (WS-I-2) in den Mischer (M-I-1) so eingespeist, dass sich das vorgegebene Phasenverhältnis Waschsäure zu DNT, einstellt. Nach Phasentrennung der Waschemulsion in dem zur Extraktionsstufe gehörenden Phasentrennapparat (S-I-1) wird die abgetrennte Waschsäure (WSR-I-1) in den zu dieser Stufe gehörenden Mischer (M-I-1) zurückgeführt. Die überschüssige Waschsäure (WSR-I-1) wird direkt oder nach Aufkonzentrierung in die Nitrierung zurückgeführt.

Das im Scheider (S-I-1) abgetrennte, einmal gewaschene DNT (DNT-I-1) wird in die nachfolgende Extraktionsstufe (WS-I-2)(n=2) zusammen mit der im Kreis geführten Waschsäure (WSR-I-2) aus dem Phasentrennapparat (S-I-2) zusammen mit der überschüssigen Waschsäure (WSR-I-3) aus der nachfolgenden Extraktionsstufe (WS-I-3) in den Mischer (M-I-2) so eingespeist, dass sich das vorgegebene Phasenverhältnis Waschsäure zu DNT einstellt. Nach Phasentrennung der Waschemulsion in dem zu dieser Extraktionsstufe gehörenden Phasentrennapparat (S-I-2) wird die abgetrennte Waschsäure (WSR-I-2) in den zur Stufe gehörenden Mischer (M-I-2) zurückgeführt. Die überschüssige Waschsäure (MSR-I-2) wird in die vorgeschaltete Extraktionsstufe (WS-I-1) eingespeist. Das im Phasentrennapparat (S-I-2) abgetrennte, zweimal gewaschene DNT (DNT-I-2) wird in die nächste Extraktionsstufe (WS-I-3)(n=3) für eine dritte Wäsche transferiert. Die Anzahl der Extraktionsststufen kann vorzugsweise bis zu vier (n=4) betragen. In die letzte Extraktionsstufe (WS-I-4) wird als Waschmedium (W-I-1) Frischwasser oder bevorzugt das Brüdenkondensat aus der Aufkonzentrierung der Waschsäure aus der Waschstufe (WS-I-1) in der Menge eingespeist, dass die aus (S-I-1) abgetrennte Waschsäure einen Gesamtsäuregehalt von 20-40% aufweist.

Das vorgewaschene DNT (DNT-I-n) aus Waschschritt (WS-I) wird im Waschschritt (WS-II), in der ersten Extraktionsstufe (WS-II-1) (m=1) zusammen mit der im Kreis geführten Waschsäure (WSR-II-1) aus dem Phasentrennapparat (S-II-1) zusammen mit der überschüssigen Waschsäure (WSR-II-2) aus der nachfolgenden Extraktionsstufe (WS-II-2) in den Mischer (M-II-1) so eingespeist, dass sich das vorgegebene Phasenverhältnis Waschsäure zu DNT einstellt. Nach Phasentrennung der Waschemulsion in dem zur Waschstufe
gehörenden Phasentrennapparat (S-II-1) wird die abgetrennte Waschsäure (WSR-II-1) in den zu dieser Stufe gehörenden Mischer (M-II-1) zurückgeführt. Die überschüssige Waschsäure (WSR-II-1) wird direkt oder nach Rückgewinnung des darin gelösten Produktes (DNT) einer abschließenden biologischen Nachbehandlung zugeführt.

Das im Phasentrennapparat (S-II-1) abgetrennte, einmal gewaschene DNT (DNT-II-1) wird in die nachfolgende Extraktionsstufe (WS-II-2) (m=2) zusammen mit der im Kreis geführten Waschsäure (WSR-II-2) aus dem Phasentrennapparat (S-II-2) und der überschüssigen Waschsäure (WSR-II-3) aus der nachfolgenden Waschstufe (WS-II-3) in den Mischer (M-II-2) so eingespeist, dass sich das vorgegebene Phasenverhältnis Waschsäure zu DNT einstellt. Nach Phasentrennung der Waschemulsion in dem zu dieser Extraktionsstufe gehörenden Phasentrennapparat (S-II-2) wird die abgetrennte Waschsäure (WSR-II-2) in den zu dieser Stufe gehörenden Mischer (M-II-2) zurückgeführt. Die überschüssige Waschsäure (MSR-II-2) wird in die vorgeschaltete Extraktionsstufe (WS-II-1) eingespeist. Das im Phasentrennapparat (S-II-2) abgetrennte, zweimal gewaschene DNT (DNT-II-2) wird in die nächste Extraktionsstufe (WS-II-3)(m=3) für eine dritte Wäsche transferiert. Die Anzahl der Extraktionsstufen kann vorzugsweise bis zu vier (m=4) betragen. In die letzte Extraktionsstufe (WS-II-4) wird als Waschmedium (W-II-1) Frischwasser in der Menge eingespeist, dass die aus (S-II-1) abgetrennte Waschsäure einen pH-Wert von bevorzugt 0-3 aufweist. Durch Zusatz von Salpetersäure oder Waschsäure (WSR-I-1) kann der pH-Wert in der ersten Waschstufe (WS-II-1) zusätzlich auf den optimalen Betriebswert nachjustiert werden.

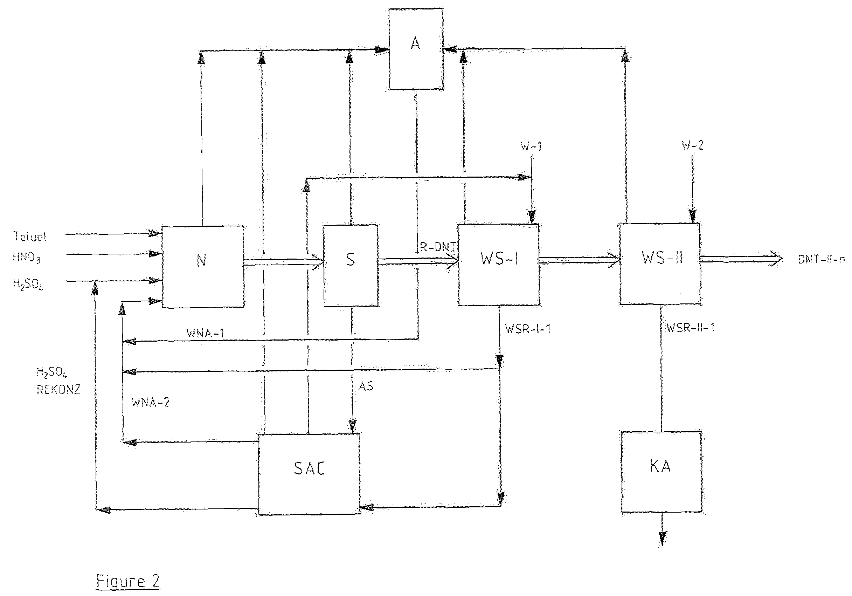

Figur 2 zeigt eine schematische Darstellung einer erfindungsgemäßen Produktionsanlage zur Nitrierung von Toluol zu DNT mit nachfolgender erfindungsgemäßer Wäsche des Roh-DNT in zwei Schritten gemäß einer bevorzugten Ausführungsform der Erfindung.

Das in der Nitriereinheit (N) durch eine zweistufige kontinuierliche isotherme oder adiabatische Nitrierung von Toluol in einem Gemisch aus Schwefel- und Salpetersäure im Gegenstrom gebildete Dinitrotoluol-Isomerengemisch wird nach Trennung der Nitrieremulsion im Phasentrennapparat (Scheider (S)) in eine erfindungsgemäße Wäsche mit zwei Waschschritten (WS-I) und (WS-II) eingespeist. In Waschschritt (WS-I) werden die im rohen DNT gelösten Mineralsäuren Schwefelsäure, Salpetersäure und Stickstoffoxide ausgewaschen. Die in diesem Waschschritt aus der ersten Waschstufe (WS-I-1) abgetrennte Waschsäure (WSR-I-1) mit einem Gesamtsäuregehalt von 20 - 40 Gew.-% wird direkt in die Nitriereinheit (N) oder nach weiterem Aufkonzentrieren als (WNA-2) in die Nitrierung zurückgeführt. Das Brüdenkondensat aus der Aufkonzentrierung der Waschsäure (WRS-I-1) und/oder der Nitrierendsäure wird in den Waschschritt (WS-I) als Waschwasser (W-1) zurückgeführt.

Im zweiten Waschschritt (WS-II) werden die im vorgewaschenen DNT aus (WS-I) noch verbliebenen Reste an Mineralsäuren derart ausgewaschen, dass eine Waschsäure (WSR-II-1) mit einem pH-Wert im Bereich von vorzugsweise 0-3 resultiert. Zur Ergänzung der abgetrennten Waschsäuremenge wird in (WS-II) Frischwasser zugesetzt. Diese Waschsäure aus Waschschritt II (WS-II) wird nach Einstellen auf einen pH-Wert von 6-8 direkt oder auch nach Rückgewinnung des darin gelösten DNT einer biologischen Nachbehandlung in einer Kläranlage (KA) unterworfen.

Die in der Scheideeinheit (S) erhaltenen Nitrierendsäure wird in einer Aufkonzentrieranlage für Schwefelsäure (SAC) wieder zu Schwefelsäure (bis zu 96%) aufkonzentriert und in die Nitrierung zurückgeführt. Die in der Abgasbehandlung (A) aus den gesammelten, Stickstoffoxide enthaltenden Abgasen aus Nitrierung und Wäsche zurück gewonnene Salpetersäure (WNA-1) wird zusammen mit der aus Waschschritt (WS-I) anfallenden Waschsäure (WSR-I-1) oder, nach Aufkonzentration, als (WNA-2) gleichfalls in die Nitrierung zurückgeführt.

Die vorliegende Erfindung wird an Hand des nachfolgenden Ausführungsbeispiels verdeutlicht.

### Beispiele

### Beispiel 1 (Stand der Technik)

4500 kg/h DNT mit einem Restgehalt an 0,94 Gew.-% Schwefelsäure, 1,43 Gew.-% Salpetersäure und 1,15 Gew.-% Stickstoffdioxid wurden nach Abtrennung der Nitrierendsäure in einer sauren Wäsche (Waschschritt I) in 2 Stufen im Gegenstrom gewaschen. In der ersten Extraktionsstufe wurde mit einer Waschsäure mit 7,58 Gew.-% Schwefelsäure, 19,52 Gew.-% an Salpetersäure und ca. 0,45 Gew.-% an Stickstoffoxiden (als HNO2) im Phasenverhältnis DNT zu Waschsäure von 1 : 1 gewaschen. In der zweiten Extraktionsstufe des Waschschrittes I wurde gleichfalls im Phasenverhältnis 1 : 1 mit einer Waschsäure mit maximal 0,5 Gew.-% Schwefelsäure, ca. 6 Gew.-% Salpetersäure und ca. 0,15 Gew.-% an Stickstoffoxiden gewaschen. Die Menge des zugesetzten Wassers bzw. Kondensates aus der weiteren Aufkonzentrierung der Waschsäure (im vorliegenden Beispiel ca. 450 I/h), das auf der letzten Extraktionsstufe eingespeist wurde, ist so gewählt, dass die Konzentration der Waschsäure in der ersten Extraktionsstufe der sauren Wäsche, im Kontakt mit dem zu waschenden Roh-DNT, die festgelegte Säurestärke und Dichte nicht überschreitet. Zur Aufrechterhaltung des vorgegebenen Phasenverhältnisses wurde die im Scheider (Phasentrennapparat) der jeweiligen Extraktionsstufe abgetrennte Waschsäure im Kreis geführt und nur der Überschuss wurde in die vorgelagerte Waschstufe transferiert bzw. ausgeschleust.

Die aus der sauren Wäsche ausgeschleuste Waschsäure mit einem Gesamtsäuregehalt von 27,55 40 Gew.-% wurde nach weiterer Aufkonzentrierung in die Nitrierung zurückgeführt.

Das DNT aus der sauren Wäsche, das fast frei von den Mineralsäuren war, wurde in der alkalischen Wäsche von den Resten der im vorgewaschenen DNT noch gelösten bzw. mitgerissenen Waschsäure und den Nitrophenolen und Nitrokresolen befreit.

Ca. 4350 kg an vorgewaschenem DNT aus Waschschritt I (saure Wäsche) mit einem Restgehalt von maximal 100 ppm an Schwefelsäure und maximal 3000 ppm an Salpetersäure/Stickstoffoxiden wurden in einem alkalischen Waschschritt (Alkaliwäsche) in Gegenwart einer Base (Soda) im Phasenverhältnis 1 : 1 in einer Stufe gewaschen, wobei sich ein pH von 9-10 einstellt. Nach Phasentrennung der Waschemulsion aus der Alkaliwäsche wurde das abgetrennte DNT noch in einer einstufigen Neutralwäsche, ebenfalls im Phasenverhältnis 1:1, von den mitgerissenen Spuren aus der Alkaliwäsche durch Zugabe von Frischwasser gewaschen. Nach Phasentrennung wird das in der Neutralwäsche abgetrennte Waschwasser in die Alkaliwäsche als Waschwasser eingespeist. Zur Aufrechterhaltung der vorgegebenen Phasenverhältnisse in den beiden Waschschritten (Alkaliwäsche und Neutralwäsche) wurde das im Scheider der jeweiligen Waschstufe abgetrennte Waschwasser im Kreis geführt und nur der Überschuss wurde von der Neutralwäsche in die Alkaliwäsche transferiert bzw. aus der Alkaliwäsche ausgeschleust.

Die aus der Alkaliwäsche ausgeschleuste alkalische Waschlauge mit einem pH von 9-10, einem Gehalt von 40 ppm Sulfat, 580 ppm Nitrat, 2500 ppm Nitrit, 990 ppm DNT und 740 ppm Trinitrokresole wurde direkt in eine Thermolyse bei 290°C und 90 bar, zum Abbau der Nitrophenole und Nitrokresole sowie weiterer Nitroverbindungen und des gelösten DNT eingespeist. Das aus der Thermolyse ablaufende Wasser, in dem die Nitrophenole und Nitrokresole und das gelösten DNT zerstört wurden, wurde vor Abgabe in den Vorfluter noch einer biologischen Nachbehandlung in einer Kläranlage unterworfen.

### Beispiel 2 (erfindungsgemäß)

4500 kg/h DNT mit einem Restgehalt an 0,94 Gew.-% Schwefelsäure, 1,43 Gew.-% Salpetersäure und 1,15 Gew.-% Stickstoffdioxid wurden nach Abtrennung der Nitrierendsäure im Waschschritt I (WS-I) in 2 Stufen im Gegenstrom gewaschen. In der ersten Extraktionsstufe (WS-I-1) wurde mit einer Waschsäure mit 7,58 Gew.-% Schwefelsäure, 19,52 Gew.-% Salpetersäure und ca. 0,45 Gew.-% an Stickstoffoxiden (als HNO2) im Phasenverhältnis DNT zu Waschsäure von 1 : 1 gewaschen. In der zweiten Extraktionsstufe (WS-I-2) des Waschschrittes I wurde gleichfalls im Phasenverhältnis 1 : 1 mit einer Waschsäure mit maximal 0,5 Gew.-% Schwefelsäure, ca. 6 Gew.-% Salpetersäure und ca. 0,15 Gew.-% Stickstoffoxiden gewaschen. Die Menge des zugesetzten Wassers bzw. Kondensates aus der weiteren Aufkonzentrierung der Waschsäure (im vorliegenden Beispiel ca. 450 I/h), das in die letzte Extraktionsstufe eingespeist wurde, ist so gewählt, dass die Konzentration der Waschsäure in der ersten Extraktionsstufe (WS-I-1) im Kontakt mit dem zu waschenden Roh-DNT die festgelegte Säurestärke und Dichte nicht überschreitet. Zur Aufrechterhaltung des vorgegebenen Phasenverhältnisses wurde die im Scheider (Phasentrennapparat) der jeweiligen Extraktionsstufe abgetrennte Waschsäure im Kreis geführt und nur der Überschuss wurde in die vorgelagerte Waschstufe transferiert bzw. ausgeschleust.

Die im Waschschritt (WS-I) ausgeschleuste Waschsäure mit einem Gesamtsäuregehalt von 27,55 Gew.-% wurde nach weiterer Aufkonzentrierung in die Nitrierung zurückgeführt.

Das im Waschschritt (WS-I) von den Mineralsäuren fast vollständig befreite, vorgewaschene DNT wurde in Waschschritt II (WS-II) von den Resten der noch im DNT gelösten bzw. mitgerissenen Waschsäure aus Waschschritt I (WS-I)befreit.

Ca. 4350 kg an vorgewaschenem DNT aus Waschschritt I (WS-I) mit einem Restgehalt von maximal 100 ppm Schwefelsäure und maximal 2000 ppm Salpetersäure/Stickstoffoxide wurden in zwei Stufen im Gegenstrom gewaschen. In der ersten Extraktionsstufe des Waschschritts II (WS-II) wurde mit einer Waschsäure mit einem pH von 0-3, besonders bevorzugt von 0,8-1,2, mit einem Phasenverhältnis DNT : Waschsäure von 1 : 2 gewaschen. In der zweiten Extraktionsstufe des Waschschrittes II (WS-II) wurde gleichfalls im Phasenverhältnis 1 : 2 mit einer Waschsäure, die nur noch Spuren an Mineralsäure, vor allen Salpetersäure aufwies, gewaschen. Die Menge des zugesetzten Frischwassers (im vorliegenden Beispiel ca. 2000 I/h), das in die letzte Extraktionsstufe (WS-II-n) (n=2) eingespeist wurde, wurde so gewählt, dass für den pH-Wert in der ersten Extraktionsstufe (WS-II-1), in der das DNT aus dem Waschschritt I (WS-I) extrahiert wird, der festgelegte optimale Wert im Bereich von 0,8-1,2 eingehalten wurde. Zur Aufrechterhaltung der vorgegebenen Phasenverhältnisse in den Extraktionsstufen wurde die im Scheider der jeweiligen Extraktionsstufe abgetrennte Waschsäure im Kreis geführt, und nur der Überschuss wurde in die vorgelagerte Stufe transferiert bzw. ausgeschleust.

Die in Waschschritt II (WS-II) aus der ersten Extraktionsstufe (WS-II-1) ausgeschleuste Waschsäure mit einem pH-Wert von 1,0, einem Gehalt von 85 ppm Schwefelsäure, 1800 ppm Salpetersäure, 40 ppm Stickstoffoxide, 980 ppm DNT, kleiner 1 ppm Trinitrokresole und kleiner 10 ppm Nitrobenzoesäuren wurde direkt in eine biologische Behandlung eingespeist. Das nach dieser biologischen Behandlung erhaltene Abwasser erfüllten alle Anforderungen an die Toxizität, die in der Abwasserverordnung der Bundesrepublik Deutschland festgelegt sind.

## Patentansprüche

1. Verfahren zur Wäsche eines bei der Nitrierung von Toluol nach Abtrennung der Nitriersäure anfallenden Rohgemischs enthaltend Dinitrotoluol, Salpetersäure, Stickstoffoxide und Schwefelsäure umfassend zwei Waschschritte (WS-I) und (WS-II), wobei
i) in einem ersten, im Gegenstrom durchgeführten Waschschritt (WS-I) das Rohgemisch in einer mindestens zwei Extraktionsstufen umfassenden Wäsche mit einer Salpetersäure, Stickstoffoxide und Schwefelsäure enthaltenden Waschsäure 1 extrahiert wird, wobei der letzten Extraktionsstufe (WS-1-n) des ersten Waschschrittes (WS-I) Frischwasser oder eine Waschsäure mit einem Gesamtsäuregehalt von 0,2 bis 1,5 Gew.-% zugeführt wird und die aus der ersten Extraktionsstufe (WS-I-1) des ersten Waschschrittes (WS-I)abgeführte Waschsäure einen Gesamtsäuregehalt von 20 bis 40 Gew.-% aufweist, wobei ein vorgewaschenes Rohgemisch erhalten wird,
ii) in einem zweiten, im Gegenstrom durchgeführten Waschschritt (WS-II) das vorgewaschene Rohgemisch enthaltend Dinitrotoluol in einer mindestens zwei Extraktionsstufen umfassenden Wäsche mit einer Waschsäure II extra-hiert wird, wobei der letzten Extraktionsstufe (WS-II-m) des zweiten Waschschrittes (WS-II) Frischwasser zugeführt wird und die aus der ersten Extraktionsstufe (WS-II-1) des zweiten Waschschrittes (WS-II) abgeführte Waschsäure einen pH-Wert von kleiner oder gleich 4 aufweist, wobei ein von Salpetersäure, Schwefelsäure und Stickstoffoxiden im wesentlichen freies, Dinitrotoluol enthaltendes Gemisch erhalten wird, das aus der letzten Extraktionsstufe (WS-II-m) des zweiten Waschschritts (WS-II) abgezogen wird und maximal 300 ppm Salpetersäure und Stickstoffoxide und maximal 3 ppm Sulfat aufweist.

2. Verfahren nach Anspruch 1, **dadurch gekennzeichnet, dass** der erste Waschschritt (WS-I) 2 bis 4 Extraktionsstufen umfasst.

3. Verfahren nach Anspruch 1 oder 2, **dadurch gekennzeichnet, dass** der zweite Waschschritt (WS-II) 2 bis 4 Extraktionsstufen umfasst.

4. Verfahren nach einem der Ansprüche 1 bis 3, **dadurch gekennzeichnet, dass** die aus der ersten Extraktionsstufe (WS-II-1) des zweiten Waschschrittes (WS-II) abgeführte Waschsäure einen pH-Wert von 0 bis 3 aufweist.

5. Verfahren nach einem der Ansprüche 1 bis 4, **dadurch gekennzeichnet, dass** die aus der ersten Extraktionsstufe (WS-II-1 des zweiten Waschschrittes (WS-II) abgeführte Waschsäure einen pH-Wert von 0,5 bis 2 aufweist.

6. Verfahren nach einem der Ansprüche 1 bis 5, **dadurch gekennzeichnet dass** der letzten Extraktionsstufe (WS-I-n) des ersten Waschschrittes (WS-I) Wasser oder eine Waschsäure mit einem Gesamtsäuregehalt von 0,2 bis 1,5 Gew.-% zugeführt wird.

7. Verfahren nach einem der Ansprüche 1 bis 6, **dadurch gekennzeichnet, dass** der letzten Extraktionsstufe (WS-II-n) des zweiten Waschschrittes (WS-II) Wasser zugeführt wird.

8. Verfahren nach einem der Ansprüche 1 bis 7, **dadurch gekennzeichnet, dass**
I) in der ersten Extraktionsstufe (WS-I-1) des ersten Waschschritts (WS-I) das Rohgemisch enthaltend Dinitrotoluol, Salpetersäure, Stickstoffoxide und Schwefelsäure in einen ersten Mischer (M-I-1 zusammen mit im Kreis geführter Waschsäure (WSR-I-1) aus einem zu dieser ersten Extraktionsstufe gehörenden ersten Phasentrennapparats (S-I-1) und überschüssiger Waschsäure (WSR-I-2) aus der nachfolgenden Extraktionsstufe (WS-I-2) eingespeist wird, die in dem Mischer gebildete Waschemulsion in dem ersten Phasentrennapparat (S-I-1) in die Waschsäure (WSR-I-1) und einmal gewaschenes Rohgemisch (DNT-I-1) aufgetrennt, und
II) das einmal gewaschene Rohgemisch (DNT-I-1) in der nachfolgenden Extraktionsstufe (WS-I-2) des ersten Waschschritts (WS-I) in einen zu dieser Extraktionsstufe gehörenden zweiten Mischer (M-I-2) zusammen mit der im Kreis geführten Waschsäure (WSR-I-2) aus einem zu dieser Extraktionsstufe gehörenden zweiten Phasentrennapparat (S-I-2) und, wenn der erste Waschschritt (WS-I) mehr als 2 Extraktionsstufen umfasst, mit überschüssiger Waschsäure (WSR-I-3) aus der nachfolgenden Extraktionsstufe (WS-I-3) oder, wenn der erste Waschschritt genau 2 Extraktionsstufen umfasst, mit Frischwasser oder Waschsäure eingespeist wird.

9. Verfahren nach einem der Ansprüche 1 bis 8, **dadurch gekennzeichnet, dass**
I) in der ersten Extraktionsstufe (WS-II-1) des zweiten Waschschritts (WS-II) das vorgewaschene Rohgemisch enthaltend Dinitrotoluol in einen zu dieser Extraktionsstufe gehörenden ersten Mischer (M-II-1) zusammen mit im Kreis geführter Waschsäure (WSR-II-1) aus einem zu dieser Extraktionsstufe gehörenden ersten Phasentrennapparat (S-II-1) und überschüssiger Waschsäure (WSR-II-2) aus der nachfolgenden Extraktionsstufe (WS-II-2) eingespeist wird, die in dem ersten Mischer gebildete Waschemulsion in dem ersten Phasentrennapparat (S-II-1) in die Waschsäure (WSR-II-1) und einmal gewaschenes Rohgemisch (DNT-II-1 aufgetrennt, und
II) das einmal gewaschene Rohgemisch (DNT-II-1) in der nachfolgenden Extraktionsstufe (WS-II-2) des zweiten Waschschritts (WS-II) in einen zu dieser Extraktionsstufe gehörenden zweiten Mischer (M-II-2) zusammen mit der im Kreis geführten Waschsäure (WSR-II-2) aus einem zu dieser zweiten Extraktionsstufe gehörenden zweiten Phasentrennapparat (S-II-2) und, wenn der zweite Waschschritt (WS-II) mehr als 2 Extraktionsstufen umfasst, mit überschüssiger Waschsäure (WSR-I-3) aus der nachfolgenden Extraktionsstufe (WS-I-3) oder, wenn der Waschschritt genau 2 Extraktionsstufen umfasst, mit Frischwasser eingespeist wird.

10. Verfahren nach einem der Ansprüche 1-9, **dadurch gekennzeichnet, dass** das Volumenverhältnis zwischen Dinitrotoluol enthaltendem Rohgemisch und Waschsäure in den Extraktionsstufen der Waschschritte (WS-I) und (WS-II) von 1:4 bis 10 : 1 beträgt.

11. Verfahren nach einem der Ansprüche 1-10, **dadurch gekennzeichnet, dass** das Volumenverhäitnis von Dinitrotoluol enthaltendem Rohgemisch zu Frischwasser in der letzen Extraktionsstufe (WS-II-n) des zweiten Waschschrittes (WS-II) von 1 : 2 bis 15 : 1 beträgt.

12. Verfahren nach einem der Ansprüche 1 bis11 **dadurch gekennzeichnet, dass** die aus der letzten Extraktionsstufe (WS-II-n) des zweiten Waschschritts (WS-II) ein Dinitrotoluol enthaltendes Gemisch abgezogen wird, welches insgesamt maximal 800 ppm Nitrokresole und Nitrophenolen und maximal 600 ppm Nitrobenzoesäuren aufweist.

13. Verfahren nach einem der Ansprüche 1 bis 12, **dadurch gekennzeichnet, dass** die aus der ersten Extraktionsstufe (WS-II-1) des zweiten Waschschritts (WS-II) abgeführte Waschsäure einen Gehalt an Sulfat von maximal 100 ppm, einem Gehalt an Salpetersäure von maximal 2000 ppm und einem Gehalt an Stickstoffoxiden (berechnet als HNO₂) von maximal 50 ppm aufweist.

14. Verfahren nach Anspruch 1 bis 13 **dadurch gekennzeichnet, dass** die aus der ersten Extraktionsstufe (WS-II-1) des zweiten Waschschritts (WS-II) abgeführte Waschsäure einen Gehalt an Nitrokresolen und Nitrophenolen von insgesamt kleiner 5 ppm und einen Gehalt an Nitrobenzoesäuren von kleiner als 50 ppm aufweist.

15. Verfahren nach Anspruch 13 oder14 **dadurch gekennzeichnet, dass** die aus der ersten Extraktionsstufe (WS-II-1) des zweiten Waschschritts (WS-II) abgeführte Waschsäure neutralisiert und, gegebenenfalls nach Abtrennung von darin gelöstem Dinitrotoluol durch Extraktion und Abtrennung des Extraktionsmittels, ohne weitere Vorbehandlung der biologischen Behandlungsstufe einer Kläranlage zugeführt wird.

## Claims

1. A process for scrubbing a crude mixture comprising dinitrotoluene, nitric acid, nitrogen oxides and sulfuric acid obtained in the nitration of toluene after the nitrating acid has been separated off, which comprises two scrubbing steps (SS-I) and (SS-II) , wherein
i) in a first scrubbing step (SS-I) carried out in countercurrent, the crude mixture is extracted with a scrubbing acid I comprising nitric acid, nitrogen oxides and sulfuric acid in a scrub comprising at least two extraction stages, where fresh water or a scrubbing acid having a total acid content of from 0.2 to 1.5% by weight is fed to the last extraction stage (SS-I-n) of the first scrubbing step (SS-I) and the scrubbing acid discharged from the first extraction stage (SS-I-1) of the first scrubbing step (SS-I) has a total acid content of from 20 to 40% by weight and a prescrubbed crude mixture is obtained,
ii)in a second scrubbing step (SS-II) carried out in countercurrent, the prescrubbed crude mixture comprising dinitroluene is extracted with a scrubbing acid II in a scrub comprising at least two extraction stages, where fresh water is fed to the last extraction stage (SS-II-m) of the second scrubbing step (SS-II) and the scrubbing acid discharged from the first extraction stage (SS-II-1) of the second scrubbing step (SS-II) has a pH of less than or equal to 4 and a mixture comprising dinitrotoluene which is essentially free of nitric acid, sulfuric acid and nitrogen oxides is obtained, which mixture is taken off from the last extraction stage (SS-II-m) of the second scrubbing step (SS-II) and comprises not more than 300 ppm of nitric acid and nitrogen oxides and not more than 3 ppm of sulfate.

2. The process according to claim 1, wherein the first scrubbing step (SS-I) comprises from 2 to 4 extraction stages.

3. The process according to claim 1 or 2, wherein the second scrubbing step (SS-II) comprises from 2 to 4 extraction stages.

4. The process according to any of claims 1 to 3, wherein the scrubbing acid discharged from the first extraction stage (SS-II-1) of the second scrubbing step (SS-II) has a pH of from 0 to 3.

5. The process according to any of claims 1 to 4, wherein the scrubbing acid discharged from the first extraction stage (SS-II-1) of the second scrubbing step (SS-II) has a pH of from 0.5 to 2.

6. The process according to any of claims 1 to 5, wherein water or a scrubbing acid having a total acid content of from 0.2 to 1.5% by weight is fed to the last extraction stage (SS-I-n) of the first scrubbing step (SS-I).

7. The process according to any of claims 1 to 6, wherein water is fed to the last extraction stage (SS-II-n) of the second scrubbing step (SS-II).

8. The process according to any of claims 1 to 7, wherein
I) in the first extraction stage (SS-I-1) of the first scrubbing step (SS-I), the crude mixture comprising dinitrotoluene, nitric acid, nitrogen oxides and sulfuric acid is fed into a first mixer (M-I-1) together with circulated scrubbing acid (SA-I-1) from a first phase separation apparatus (S-I-1) belonging to this first extraction stage and excess scrubbing acid (SA-I-2) from the subsequent extraction stage (SS-I-2), the scrubbing emulsion formed in the mixer is separated in the first phase separation apparatus (S-I-1) into the scrubbing acid (SA-I-1) and crude mixture which has been scrubbed once (DNT-I-1) and
II) the crude mixture which has been scrubbed once (DNT-I-1) is, in the subsequent extraction stage (SS-I-2) of the first scrubbing step (SS-I), fed into a second mixer (M-I-2) belonging to this extraction stage together with the circulated scrubbing acid (SA-I-2) from a second phase separation apparatus (S-I-2) belonging to this extraction stage and, if the first scrubbing step (SS-I) comprises more than 2 extraction stages, with excess scrubbing acid (SA-I-3) from the subsequent extraction stage (SS-I-3) or, if the first scrubbing step comprises precisely 2 extraction stages, with fresh water or scrubbing acid.

9. The process according to any of claims 1 to 8, wherein
I) in the first extraction stage (SS-II-1) of the second scrubbing step (SS-II), the prescrubbed crude mixture comprising dinitrotoluene is fed into a first mixer (M-II-1) belonging to this extraction stage together with circulated scrubbing acid (SA-II-1) from a first phase separation apparatus (S-II-1) belonging to this extraction stage and excess scrubbing acid (SA-II-2) from the subsequent extraction stage (SS-II-2), the scrubbing emulsion formed in the first mixer is separated in the first phase separation apparatus (S-II-1) into the scrubbing acid (SA-II-1) and crude mixture which has been scrubbed once (DNT-II-1) and
II) the crude mixture which has been scrubbed once (DNT-II-1) is, in the subsequent extraction stage (SS-II-2) of the second scrubbing step (SS-II), fed into a second mixer (M-II-2) belonging to this extraction stage together with the circulated scrubbing acid (SA-II-2) from a second phase separation apparatus (S-II-2) belonging to this second extraction stage and, if the second scrubbing step (SS-II) comprises more than 2 extraction stages, with excess scrubbing acid (SA-I-3) from the subsequent extraction stage (SS-I-3) or, if the scrubbing step comprises precisely 2 extraction stages, with fresh water.

10. The process according to any of claims 1-9, wherein the volume ratio of dinitrotoluene-comprising crude mixture to scrubbing acid in the extraction stages of scrubbing steps (SS-I) and (SS-II) is from 1:4 to 10:1.

11. The process according to any of claims 1-10, wherein the volume ratio of dinitrotoluene-comprising crude mixture to fresh water in the last extraction stage (SS-II-n) of the second scrubbing step (SS-II) is from 1:2 to 15:1.

12. The process according to any of claims 1 to 11, wherein a dinitrotoluene-comprising mixture comprising a total of not more than 800 ppm of nitrocresols and nitrophenols and not more than 600 ppm of nitrobenzoic acids is taken off from the last extraction stage (SS-II-n) of the second scrubbing step (SS-II).

13. The process according to any of claims 1 to 12, wherein the scrubbing acid discharged from the first extraction stage (SS-II-1) of the second scrubbing step (SS-II) has a content of sulfate of not more than 100 ppm, a content of nitric acid of not more than 2000 ppm and a content of nitrogen oxides (calculated as HNO₂) of not more than 50 ppm.

14. The process according to any of claims 1 to 13, wherein the scrubbing acid discharged from the first extraction stage (SS-II-1) of the second scrubbing step (SS-II) has a total content of nitrocresols and nitrophenols of less than 5 ppm and a content of nitrobenzoic acids of less than 50 ppm.

15. The process according to claim 13 or 14, wherein the scrubbing acid discharged from the first extraction stage (SS-II-1) of the second scrubbing step (SS-II) is neutralized and, optionally after removal of dinitrotoluene dissolved therein by extraction and removal of the extractant, fed without further pretreatment to the biological treatment stage of a water treatment plant.

## Revendications

1. Procédé de lavage d'un mélange brut formé lors de la nitration de toluène après séparation de l'acide sulfonitrique, contenant du dinitrotoluène, de l'acide nitrique, des oxydes d'azote et de l'acide sulfurique, comprenant deux étapes de lavage (WS-I) et (WS-II), selon lequel
i) lors d'une première étape de lavage (WS-I) réalisée à contre-courant, le mélange brut est extrait dans un lavage comprenant au moins deux étapes d'extraction avec un acide de lavage I contenant de l'acide nitrique, des oxydes d'azote et de l'acide sulfurique, de l'eau fraîche ou un acide de lavage ayant une teneur totale en acide de 0,2 à 1,5 % en poids étant introduit dans la dernière étape d'extraction (WS-I-n) de la première étape de lavage (WS-I), et l'acide de lavage déchargé de la première étape d'extraction (WS-I-1) de la première étape de lavage (WS-I) présentant une teneur totale en acide de 20 à 40 % en poids, un mélange brut prélavé étant obtenu,
ii) lors d'une seconde étape de lavage (WS-II) réalisée à contre-courant, le mélange brut prélavé contenant du dinitrotoluène est extrait dans un lavage comprenant au moins deux étapes d'extraction avec un acide de lavage II, de l'eau fraîche étant introduite dans la dernière étape d'extraction (WS-II-m) de la seconde étape de lavage (WS-II), et l'acide de lavage déchargé de la première étape d'extraction (WS-II-1) de la seconde étape de lavage (WS-II) présentant un pH inférieur ou égal à 4, un mélange contenant du dinitrotoluène, essentiellement exempt d'acide nitrique, d'acide sulfurique et d'oxydes d'azote, étant obtenu, qui est soutiré de la dernière étape d'extraction (WS-II-m) de la seconde étape de lavage (WS-II), et comprend au plus 300 ppm d'acide nitrique et d'oxydes d'azote et au plus 3 ppm de sulfate.

2. Procédé selon la revendication 1, **caractérisé en ce que** la première étape de lavage (WS-I) comprend 2 à 4 étapes d'extraction.

3. Procédé selon la revendication 1 ou 2, **caractérisé en ce que** la seconde étape de lavage (WS-II) comprend 2 à 4 étapes d'extraction.

4. Procédé selon l'une quelconque des revendications 1 à 3, **caractérisé en ce que** l'acide de lavage déchargé de la première étape d'extraction (WS-II-1) de la seconde étape de lavage (WS-II) présente un pH de 0 à 3.

5. Procédé selon l'une quelconque des revendications 1 à 4, **caractérisé en ce que** l'acide de lavage déchargé de la première étape d'extraction (WS-II-1) de la seconde étape de lavage (WS-II) présente un pH de 0,5 à 2.

6. Procédé selon l'une quelconque des revendications 1 à 5, **caractérisé en ce que** de l'eau ou un acide de lavage ayant une teneur totale en acide de 0,2 à 1, 5 % en poids est introduit dans la dernière étape d'extraction (WS-I-n) de la première étape de lavage (WS-I).

7. Procédé selon l'une quelconque des revendications 1 à 6, **caractérisé en ce que** de l'eau est introduite dans la dernière étape d'extraction (WS-II-n) de la seconde étape de lavage (WS-II).

8. Procédé selon l'une quelconque des revendications 1 à 7, **caractérisé en ce que**
I) lors de la première étape d'extraction (WS-I-1) de la première étape de lavage (WS-I), le mélange brut contenant du dinitrotoluène, de l'acide nitrique, des oxydes d'azote et de l'acide sulfurique est introduit dans un premier mélangeur (M-I-1) conjointement avec un acide de lavage (WSR-I-1) mis en circulation, provenant d'un premier appareil de séparation de phases (S-I-1) qui appartient à cette première étape d'extraction, et un acide de lavage en excès (WSR-I-2) provenant de l'étape d'extraction suivante (WS-I-2), l'émulsion de lavage formée dans le mélangeur est séparée dans le premier appareil de séparation de phases (S-I-1) en l'acide de lavage (WSR-I-1) et le mélange brut lavé à une reprise (DNT-I-1), et
II) le mélange brut lavé à une reprise (DNT-I-1) est introduit dans l'étape d'extraction suivante (WS-I-2) de la première étape de lavage (WS-I) dans un second mélangeur (M-I-2) qui appartient à cette étape d'extraction conjointement avec l'acide de lavage (WSR-1-2) mis en circulation, provenant d'un second appareil de séparation de phases (S-I-2) qui appartient à cette étape d'extraction, et, lorsque la première étape de lavage (WS-I) comprend plus de 2 étapes d'extraction, avec l'acide de lavage en excès (WSR-I-3) provenant de l'étape d'extraction suivante (WS-I-3) ou, lorsque la première étape de lavage comprend exactement 2 étapes d'extraction, avec de l'eau fraîche ou un acide de lavage.

9. Procédé selon l'une quelconque des revendications 1 à 8, **caractérisé en ce que**
I) lors de la première étape d'extraction (WS-II-1) de la seconde étape de lavage (WS-II), le mélange brut prélavé contenant du dinitrotoluène est introduit dans un premier mélangeur (M-II-1) qui appartient à cette étape d'extraction conjointement avec un acide de lavage (WSR-II-1) mis en circulation, provenant d'un premier appareil de séparation de phases (S-II-1) qui appartient à cette étape d'extraction, et un acide de lavage en excès (WSR-II-2) provenant de l'étape d'extraction suivante (WS-II-2), l'émulsion de lavage formée dans le premier mélangeur est séparée dans le premier appareil de séparation de phases (S-II-1) en l'acide de lavage (WSR-II-1) et le mélange brut lavé à une reprise (DNT-II-1), et
II) le mélange brut lavé à une reprise (DNT-II-1) est introduit dans l'étape d'extraction suivante (WS-II-2) de la seconde étape de lavage (WS-II) dans un second mélangeur (M-II-2) qui appartient à cette étape d'extraction conjointement avec l'acide de lavage (WSR-II-2) mis en circulation, provenant d'un second appareil de séparation de phases (S-II-2) qui appartient à cette seconde étape d'extraction, et, lorsque la seconde étape de lavage (WS-II) comprend plus de 2 étapes d'extraction, avec l'acide de lavage en excès (WSR-I-3) provenant de l'étape d'extraction suivante (WS-I-3) ou, lorsque l'étape de lavage comprend exactement 2 étapes d'extraction, avec de l'eau fraîche.

10. Procédé selon l'une quelconque des revendications 1 à 9, **caractérisé en ce que** le rapport volumique entre le mélange brut contenant du dinitrotoluène et l'acide de lavage dans les étapes d'extraction des étapes de lavage (WS-I) et (WS-II) est de 1:4 à 10:1.

11. Procédé selon l'une quelconque des revendications 1 à 10, **caractérisé en ce que** le rapport volumique entre le mélange brut contenant du dinitrotoluène et l'eau fraîche dans la dernière étape d'extraction (WS-II-n) de la seconde étape de lavage (WS-II) est de 1:2 à 15:1.

12. Procédé selon l'une quelconque des revendications 1 à 11, **caractérisé en ce que** le mélange contenant du dinitrotoluène qui est soutiré de la dernière étape d'extraction (WS-II-n) de la seconde étape de lavage (WS-II) comprend au total au plus 800 ppm de nitrocrésols et de nitrophénols et au plus 600 ppm d'acides nitrobenzoïques.

13. Procédé selon l'une quelconque des revendications 1 à 12, **caractérisé en ce que** l'acide de lavage déchargé de la première étape d'extraction (WS-II-1) de la seconde étape de lavage (WS-II) présente une teneur en sulfate d'au plus 100 ppm, une teneur en acide nitrique d'au plus 2 000 ppm et une teneur en oxydes d' azote (calculée sous la forme HNO₂) d' au plus 50 ppm.

14. Procédé selon les revendications 1 à 13, **caractérisé en ce que** l'acide de lavage soutiré de la première étape d'extraction (WS-II-1) de la seconde étape de lavage (WS-II) présente une teneur en nitrocrésols et en nitrophénols au total inférieure à 5 ppm et une teneur en acides nitrobenzoïques inférieure à 50 ppm.

15. Procédé selon la revendication 13 ou 14, **caractérisé en ce que** l'acide de lavage déchargé de la première étape d'extraction (WS-II-1) de la seconde étape de lavage (WS-II) est neutralisé et introduit, éventuellement après séparation du dinitrotoluène dissous dans celui-ci par extraction et séparation de l'agent d'extraction, sans prétraitement supplémentaire dans l'étape de traitement biologique d'une station d'épuration des eaux.
